# EUROPÄISCHE PATENTSCHRIFT

(11) **EP 0 529 300 B1**
(45) Veröffentlichungstag und Bekanntmachung des Hinweises auf die Patenterteilung: **14.10.1998**
(21) Anmeldenummer: 92112427.7
(22) Anmeldetag: 21.07.1992
(51) Int. Cl.: C07K 14/00, C07K 1/00, A61K 38/21

(54) **Neues rekombinantes Human-IFN-beta, Verfahren zu dessen Herstellung und dieses enthaltende pharmazeutische Zubereitungen**
Novel recombinant human-IFN-beta, process for the preparation thereof and pharmaceutical compositions comprising the same
Nouvel interféron-bêta humain, procédé de préparation et compositions pharmaceutiques le contenants

(30) Priorität: 27.08.1991 DE 4128319
(43) Veröffentlichungstag der Anmeldung: 03.03.1993
(73) Patentinhaber: Dr. Rentschler Biotechnologie GmbH, 88471 Laupheim (DE)
(72) Erfinder: Siklosi, Thomas#, W-7959 Walpertshofen# (DE); Joester, Karl-Eduard#, W-7959 Walpertshofen# (DE); Hofer, Hans#, W-7959 Walpertshofen# (DE)
(74) Vertreter: Sandmair, Kurt, Dr. Dr.

(56) Entgegenhaltungen:
- EP-A- 70 906
- EP-A- 83 069
- EP-A- 388 799
- WO-A-83/03103
- DNA, Vol. 3, No. 4 ( 1984 ), Seiten 297-308
- Journal of Biol. Chem., Vol. 262, No. 30 ( 1987 ), Seiten 14600 - 14605
- J. Biol. Chem., Vol. 263, No. 33 ( 1988 ), Seiten 17508-17515
- Eur. J. Biochem. 181 ( 1989 ), Seiten 545-553

## Beschreibung

Die Erfindung betrifft ein neues rekombinantes Fibroblasteninterferon, Verfahren zu dessen Herstellung und pharmazeutische Zubereitungen enthaltend dieses rekombinante Human-Interferon-beta (IFN-beta).

Natürlich vorkommende Interferone sind speziesspezifische Proteine, meistens Glykoproteine, die von verschiedenen Zellen nach einer Induktion mit Viren, doppelsträngiger RNA, anderen Polynucleotiden, Antigenen und Mitogenen produziert werden. Die Interferone besitzen zahlreiche biologische Aktivitäten. Die wichtigsten sind die antivirale, antiproliferative, immunmodulierende und die antizelluläre Wirkung. Bei den humanen Interferonen unterscheidet man drei Typen, die in Leukocyten, Lymphocyten, Fibroblasten und innerhalb des Immunsystems produziert werden. Sie werden als alpha-, beta- und gamma-Interferone bezeichnet.

Natürliches Human-IFN-beta wird von induzierten Humanfibroblasten-Zellkulturen produziert und aus dem Zellkulturüberstand isoliert und gereinigt. Proteine oder Polypeptide mit Human-IFN-beta-Aktivität können auch unter Verwendung der rekombinanten DNA-Technik in Mikroorganismen oder eukaryotischen Zellsystemen, wie z. B. Säugerzellkulturen, produziert werden, vgl. William E. Stewart II, The Interferon System, 2. Ausgabe, Springer-Verlag Wien, New York (1981); EP-A 41 313; W. Reiser und H. Hauser, Arzneim. Forsch./Drug. Res. 37 (1), Hr. 4 (1987), Seite 482; Mc Cormic et al., Molecular and Cellular Biology, Vol. 4, No. 1, Seite 166 (1984); EP-A-0 388 799; Chernajovsky et al., DNA, Vol. 3, No.4, Seite 297 (1984).

Natürliche und rekombinante Interferone wurden bisher mit mehr oder weniger großem Erfolg zur Behandlung von Patienten mit Tumor-, Virus-und Autoimmunerkrankungen eingesetzt. Mit verschiedenen alpha-Interferonen wurde ein Tumor-Response von 20 bis 40 % von Patienten im Frühstadium des mit AIDS verbundenen Kaposi-Sarkoms beobachtet. Nachteilig bei der Behandlung mit alpha-Interferon ist jedoch die hämatologische Intoleranz und hepatische Komplikationen, die die einsetzbare Dosis limitieren. Ein weiterer Nachteil bei der IFN-alpha-Behandlung und gleichzeitiger Verabreichung von Azidothymidin liegt darin, daß beide Substanzen eine hohe hämatologische Toxizität besitzen, so daß eine gemeinsame Verabreichung mit nicht tolerablen Nebenwirkungen verbunden ist und damit ausscheidet; vgl. Annals of Internal Medicine, Vol. 112, No. 3, 582 (1990).

Fibroblasten-Interferon (IFN-beta) besitzt viele immunologische Eigenschaften, die mit denen des IFN-alpha vergleichbar sind. Jedoch wird IFN-beta wesentlich besser toleriert und besitzt offensichtlich auch eine geringere hämatologische Toxizität als IFN-alpha in den bisher untersuchten Fällen von Nierenzellkarzinom und lymphoiden Tumoren. In in vitro Untersuchungen inhibiert IFN-beta das HIV-Virus und zeigt eine synergistische antiretrovirale Aktivität mit Azidothymidin.

Da bei längerer Verabreichung von Azidothymidin allein eine Resistenz entwickelt wird, wäre es von großem Vorteil, über Substanzen wie IFN-beta zu verfügen, die sich im Idealfall hinsichtlich der antiretroviralen Aktivität, des Wirkmechanismus und des Toxizitätsprofils von Azidothymidin unterscheiden.

Ein weiterer Nachteil der Interferone, insbesondere auch der rekombinanten, ist ihre Instabllität. Insbesondere die hochreinen Interferonpräparate für die klinische Anwendung zeigen sowohl im flüssigen als auch im lyophilisierten Zustand einen raschen Aktivitätsverlust. Für Interferone, die mittels der rekombinanten DNA-Technologie in Bakterien hergestellt werden, besteht ein weiterer Nachteil darin, daß auf diese Art produzierte Interferone in wäßrigen Lösungen nahezu unlöslich sind.

Aus dem Stand der Technik sind zahlreiche Vorschläge zur Überwindung dieser Nachteile bekannt, wobei die meisten dieser Verfahren den Zusatz verschiedenster chemischer Verbindungen zur Interferonpräparation vorschlagen, um die Löslichkeits- und/oder Stabilitätsprobleme zu beseitigen.

Alle derartige Verfahren besitzen jedoch nach wie vor die Nachteile, daß sie bei der Herstellung der Interferonpräparation einen zusätzlichen Arbeitsschritt erfordern, daß die Stabilität weiterhin nicht befriedigend ist und daß viele der Zusätze medizinisch nicht zulässig sind, weil sie entweder bekanntermaßen toxische Wirkungen besitzen oder weil ihre Toxikologie bislang ungeklärt ist, so daß derartige Zusätze von vorneherein für eine klinische Verwendung ausscheiden; vgl. EP-A 270 799, EP-A 89 245, EP-A 217 645, EP-A 215 658, WO 89/05158, WO 89/02750, EP-A 82 481, EP-A 80 879, US-PS 44 83 849 und WO 90/03784.

Die Anmelderin hat nun überraschend gefunden, daß mit einem IFN-beta, dessen Herstellung im folgenden beschrieben wird, nicht nur eine unerwartet verbesserte Antitumorwirkung erzielbar ist, sondern auch stabilere flüssige oder feste (lyophilisierte) Zubereitungen als die im Stand der Technik bekannten, hergestellt werden können.

Erfindungsgemäß werden daher ein neues rekombinantes IFN-beta mit den Merkmalen des Anspruchs 1, Verfahren zu dessen Herstellung sowie stabile pharmazeutische Zubereitungen von IFN-beta in flüssiger oder lyophilisierter bzw. fester Form zur Verfügung gestellt, die hochreines rekombinantes IFN-beta in hohen Dosen ohne irgendwelche pharmakologisch nachteiligen Zusätze enthalten. Das erfindungsgemäße IFN-beta besitzt eine höhere Antitumorwirkung, insbesondere beim Kaposi-Sarkom, als die bislang getesteten IFN-beta-Präparationen von natürlichem oder rekombinantem IFN-beta.

Erfindungsgemäß wird das neue IFN-beta mittels der rekombinanten DNA-Technik in CHO-Zellkulturen produziert und durch ein Verfahren, das aus einer Kombination von Phasenverteilung-, Affinitäts-, Metallchelat- und Size-Exclusion-Chromatographie besteht, hergestellt.

Das erfindungsgemäße rekombinante humane IFN-beta ist gekennzeichnet durch:
(a) ein bestimmtes Glykosylierungsmuster;
(b) eine hohe Volumenaktivität von > 40 x 10⁶ IU/ml;
(c) hohe Eigenstabilität;
(d) höhere klinische Wirksamkeit, insbesondere eine höhere Antitumorwirkung.

Zur Herstellung des erfindungsgemäßen IFN-beta werden transfizierte Zellen der CHO-Linie (chinese hamster ovary) mit der Laborbezeichnung BIC 8622 (Konstruktion der animalen Zellinie, vgl. Europäische Patentanmeldung Nr. 0 287 075), in üblichem Zellkulturmedium (Modified Eagles Medium (MEM) mit Earles Salzen), supplementiert mit 0 bis 5 % fötalem Kälberserum (FCS), in stationärer Kultur zur Konfluenz gebracht. Die Zellen sezernieren konstitutiv zwischen 1 x 10⁸ und 2 x 10⁹ Internationale Einheiten (IU) pro Tag und pro Liter Kulturüberstand.

Erfindungsgemäß wird das IFN-beta weiter über flüssig/flüssig Phasenextraktion angereichert, wobei wäßrige Zweiphasensysteme auf der Basis Polyalkylenglykol/Dextran oder Polyalkylenglykol/Salz zum Einsatz kommen.

Bei den Polyalkylenglykolen können Polyethylenglykole und/oder Polypropylenglykole eingesetzt werden. Als Polyethylenglykole können Polyethylenglykole mit einem MW von 1.000 bis 6.000, bevorzugt 1.200 bis 3.000, am meisten bevorzugt 1.500 bis 2.000 verwendet werden. Die Konzentration liegt im Bereich von 1 bis 30 Gew.-%, bevorzugt 2 bis 15 Gew.-%, am meisten bevorzugt 3 bis 9 Gew.-%. Als Polypropylenglykole können Polypropylenglykole mit einem MW von 1.500 bis 4.000 verwendet werden. Die verwendeten Konzentrationen entsprechen denen für die Polyethylenglykole. Als Dextran können Dextrane mit einem Molekulargewicht von 15.000 bis 5.000.000, bevorzugt 100.000 bis 1.000.000, am meisten bevorzugt 350.000 bis 550.000 verwendet werden. Die Konzentration liegt im Bereich von 1 bis 10 Gew.-%, bevorzugt 2 bis 6 Gew.-% und am meisten bevorzugt im Bereich von 4 bis 6 Gew.-%.

Als Salze können NaCl, LiCl, NaJ, KJ, Na₂SO₄, Na₂HPO₄, K₂HPO₄, K₂SO₄, NaH₂PO₄, KCl, NH₄Cl, (NH₄)₂SO₄, Na-Citrat und Na-Oxalat allein oder in Mischung verwendet werden. Die Konzentration des Salzes liegt im Bereich von 2 bis 30 Gew.-%, bevorzugt 3 bis 20 Gew.-% und am meisten bevorzugt im Bereich von 4 bis 16 Gew.-%.

Das IFN-beta enthaltende Zellkulturmedium wird zusammen mit den Bestandteilen der beschriebenen wäßrigen Phasensysteme intensiv für 5 bis 24 h, bevorzugt 8 bis 16 h, am meisten bevorzugt 10 bis 12 h bei 4 bis 25 °C, bevorzugt 10 bis 20 °C, am meisten bevorzugt 13 bis 18 °C gerührt und nach der Entmischung und Abtrennung in der Polyalkylenglykol-haltigen Oberphase angereichert.

Die Trennung der wäßrigen Phasen geschieht durch Gravitation oder durch Separation.

Die Polyalkylenglykol-haltige Oberphase enthält rekombinantes IFN-beta in einer Konzentration von 1 x 10⁸ bis 1 x 10¹⁰ IU/Liter. Die spezifische Aktivität beträgt 5 x 10⁵ bis 3 x 10⁷ IU/mg Protein. Die Ausbeuten liegen nahezu bei 100 %.

Für die sich anschließende Affinitätschromatographie wird Blau-Dextran-Sepharose ® , oder andere geeignete mit Cibacron ^{®} Blau immobilisierte Matrices wie Matrex Gel Blue A von Amicon oder Fraktogel TSK AF-Blue von Merck oder Blue-Sepharose ® 6FF von Pharmacia, verwendet.

Die aus dem Phasensystem stammende Oberphase wird mit NaCl auf eine Konzentration zwischen 0,5 und 1,0 mol/l gebracht und mit einer Flußrate von 1 bis 5 cm/min auf die Affinitätssäule aufgetragen. Nach Waschung mit PBS, pH 7, oder PBS, enthaltend 1 bis 30 Gew.-% Ethylenglykol und/oder 1 bis 15 % Propylenglykol, wird anschließend mit PBS, enthaltend 10 bis 70 Gew.-% Ethylenglykol und/oder 20 bis 50 Gew.-% Propylenglykol, eluiert.

Nach diesem Schritt weist das rekombinante IFN-beta eine Konzentration von 6 x 10⁶ bis 9 x 10⁷ IU/ml Eluat und eine spezifische Aktivität von 50 x 10⁶ bis 140 x 10⁶ IU/mg Protein auf. Die Ausbeuten liegen zwischen 70 und 90 %.

Für die Metallchelatchromatographie sind verschiedene Matrices mit chemisch identischen oder unterschiedlichen Liganden geeignet. Die zur koordinativen Bindung von rekombinantem IFN-beta geeigneten Metallionen können Cu²⁺, Zn²⁺, Co²⁺ oder Ni²⁺ Ionen sein. Die Desorption kann mittels kompetetiver Substanzen wie Imidazol, Histidin, Glycin oder NH₄Cl, Chelatbildner wie EDTA, IDA (Iminodiessigsäure), TED (Tris-Carboxymethylethylendiamin) oder durch Erniedrigung des pH-Wertes auf pH 2 bis pH 4 erfolgen.

Geeignete Trennmedien sind Immobilized Iminodiacetic Acid gekoppelt an Agarose oder an Fraktogel TSK HW-65F der Firma Pierce oder Chelating Sepharose ® FF der Firma Pharmacia oder Cellufine Chelate der Firma Amicon.

Das Chromatographiemedium wird dazu in eine geeignete Chromatographiesäule gefüllt und mit dem entsprechenden Metallkation beladen, indem man eine Lösung dieses Metallions über die Säule pumpt. Nach Äquilibrieren mit einem geeigneten Puffer, z. B. PBS, enthaltend 0,1 bis 1,0 mol/l NaCl, wird das Eluat der Affinitätschromatographie mit einer Flußrate von 0,75 bis 1,5 cm/min aufgetragen. Die Säule wird anschließend mit einem Gradienten von 0 bis 100 mmol/l Imidazol in PBS eluiert. Alternativ kann auch ein pH-Gradient von pH 7 bis pH 2, hergestellt durch Mischen der Puffer A (PBS; pH 7) und Puffer B (0,2 mol/l Glycin/HCl; pH 2), zur Elution verwendet werden. Die isokratische Elution mit PBS/Glycin/HCl-Puffern mit sinkenden pH-Werten ist ebenfalls möglich.

Nach diesem Reinigungsschritt weist das rekombinante IFN-beta eine Konzentration von 50 x 10⁶ bis 300 x 10⁶ IU/ml Eluat und eine spezifische Aktivität von 150 x 10⁶ IU/mg bis 220 x 10⁶ Protein auf. Die Ausbeuten liegen zwischen 60 und 90 %.

Für die Size-Exclusions-Chromatographie sind verschiedene Trennmedien geeignet, die einen Fraktionierbereich zwischen etwa 1.000 und etwa 600.000 Dalton aufweisen. Beispielsweise können Sephadex ® G150, G150 superfine, Sephacryl ^{R} S-200 High Resolution, Superose ^{R} 12 prep grade der Firma Pharmacia oder TSK-SW 3000 der Firma Toyo Soda verwendet werden.

Das Chromatographiemedium wird dazu in eine geeignete Chromatographiesäule gefüllt und nach Absetzen mit einem geeigneten Puffer äquilibriert. Als Puffer kommen PBS mit 0 bis 0,5 mol/l NaCl zum Einsatz. Das Eluat der Metallchromatographie wird mit einer Fließgeschwindigkeit von 0,5 bis 0,8 cm/min aufgetragen. Die Chromatographie erfolgt mit Laufpuffer PBS, enthaltend 0 bis 0,5 mol/l NaCl.

Nach diesem Reinigungsschritt weist das rekombinante IFN-beta eine Konzentration von 15 x 10⁶ bis 50 x 10⁶ IU/ml Eluat und eine spezifische Aktivität von 200 x 10⁶ bis 300 x 10⁶ IU/mg Protein auf. Die Ausbeuten liegen zwischen 60 und 80 %.

Das erhaltene Material kann anschließend direkt weiterverarbeitet werden oder bei - 20 °C gelagert werden.

Alle Reinigungsschritte werden bei einer Temperatur von 4 bis 15 °C durchgeführt.

Für den Fachmann galt zum Zeitpunkt der Anmeldung glykosyliertes und nichtglykosyliertes Interferin-beta als gleichwertig (vgl. M. Hawkins et al., Cancer Research 45 (1985), S. 5914-5920.

Die Anmelderin hat dagegen gefunden, daß das wie vorstehend beschrieben hergestellte IFN-beta einen hohen Anteil an verzweigten Kohlenhydratketten, biantennär, triantennär, triantennär mit mindestens einem Repeat und tetraantennär sowie einen hohen Gehalt an Sialinsäure und Fucose aufweisen muß, um zur Lösung des gegebenen Problems geeignet zu sein.

Es wurde gefunden, daß das erfindungsgemäße rekombinante IFN-beta einen Anteil an blantennären Oligosaccharid-Strukturen von mindestens 70 % und bevorzugt 75 % ausweist. Ein weiterer wesentlicher Bestandteil des erfindungsgemäßen rekombinanten IFN-beta ist ein Anteil an triantennären Strukturen, gegebenenfalls mit mindestens einem Repeat, die 1->4 und 1->6 verknüpft sind, wobei der Anteil an triantennären Strukturen mindestens 20 % und bevorzugt 25 % beträgt. Das erfindungsgemäße rekombinante IFN-beta enthält weiterhin einen Anteil von 0 bis 5 %, bevorzugt 0,5 - 3 % an tetraantennären Oligosaccharid-Strukturen.

Das erfindungsgemäße rekombinante IFN-beta weist in seinem gesamten Oligosaccharidanteil einen Sialinsäuregehalt von mindestens 90 % auf. Der Sialinsäureanteil setzt sich dabei aus 90 bis 100 % N-Acetylneuraminsäure und 0 bis 10 % N-Glykolylneuraminsäure zusammen.

Das erfindungsgemäße rekombinante IFN-beta enthält darüberhinaus Fucose. Obwohl der Fucosegehalt für die Stabilität offensichtlich nicht wesentlich ist, beträgt der Anteil 85 %, bevorzugt 90 % und am meisten bevorzugt > 95 %.

Die spezifischen Zuckerreste entsprechen den in den Unteransprüchen dargestellten Strukturformeln. Im Vergleich zum IFN-beta aus Fibroblasten, beispielsweise FS4-Zellen oder anderen CHO-Zellen bzw. anderen Herstellungsverfahren (Chernajovsky et al., DNA 3 (1984), 297-308; Conradt et al., J. Biol. Chem., 262 (1987), 14600-14605; Kagawa et al., J. Biol. Chem. 263, (1988), 17508-17515; Utsumi et al., Eur. J. Biochem. 181 (1989), 545-553; EP-A-0 388 799), waeist das erfindungsgemäß rekombinante IFN-beta somit eine hohe Antennärität bei gleichzeitig hohem Sialylierungsgrad auf.

Die Erfindung betrifft weiterhin pharmazeutische Zubereitungen, die rekombinantes IFN-beta mit den vorstehend beschriebenen Merkmalen in Verbindung mit bekannten und üblichen Träger- und Hilfsstoffen enthalten.

Zu den erfindunsgemäßen Zubereitungen sind auch high dosage-Zubereitungen zu zählen, d. h. Zubereitungen die pro Zubereitung zwischen etwa 18 x 10⁶ und etwa 50 x 10⁶ IU, bevorzugt etwa 20 x 10⁶ bis etwa 40 x 10⁶ IU und besonders bevorzugt etwa 25 x 10⁶ bis 30 x 10⁶ IU IFN-beta enthalten.

Als Trägerstoff kann z. B. ein Puffer auf wäßriger Basis verwendet werden, wobei der ph-Wert des Lösungsmittels im physiologisch akzeptablen, bevorzugt im neutralen Bereich, d. h. in einem Bereich von etwa 4 bis etwa 8 liegt.

Es ist nicht erforderlich, pH-Werte 〈 4 oder 〉 8 zu verwenden, um den Wirkstoff zu lösen. Ebenfalls kann auf den Zusatz von lösungsvermittelnden Substanzen, wie SDS oder anderen Detergentien, verzichtet werden.

Es werden lediglich Füllstoffe, bevorzugt ein Serumprotein, wie HSA (humanes Serumalbumin) oder andere bekannte Füllstoffe, wie PVP, verwendet.

Die erfindungsgemäße pharmazeutischen Zubereitungen können zur Behandlung von Tumoren, Viruserkrankungen, Immunopathien oder Entzündungen einschließlich rheumatischer Erkrankungen, Allergien, Psoriasis, Morbus Crohn und degenerativen Erkrankungen des Nervensystems, z. B. Multiple Sklerose, verwendet werden.

Die erforderliche Menge des zu verwendenden rekombinanten IFN-beta (im folgenden als der aktive Bestandteil bezeichnet) im Arzneimittel für die gewünschte therapeutische Wirkung hängt von der jeweiligen Verabreichungsart und von dem zu behandelnden Subjekt, sowie der jeweiligen Krankheit ab. Eine geeignete Dosis des aktiven Bestandteils zur Verabreichung am Menschen liegt zwischen etwa 0,1 x 10⁶ und 100 x 10⁶ I. E.. Die am meisten bevorzugte Dosis beträgt bei lokaler Therapie etwa 0,1 x 10⁶ bis etwa 6 x 10⁶ I. E., bei systematischer Therapie etwa 1 x 10⁶ bis 30 x 10⁶ I. E. pro Tag, gegebenenfalls mehrmals pro Tag.

Obwohl es grundsätzlich möglich ist, den aktiven Bestandteil alleine zu verabreichen, ist es vorzuziehen, daß der aktive Bestandteil in Form einer pharmazeutischen Formulierung, die den aktiven Bestandteil in einem dafür pharmazeutisch annehmbaren Trägerstoff enthält, verabreicht wird. Üblicherweise beträgt der aktive Bestandteil in einer derartigen Formulierung 1 x 10⁶ bis 50 x 10⁶ I. E./ml Wirkstofflösung. Für die topische Verabreichung beträgt der aktive Bestandteil etwa 0,1 x 10⁶ bis 10 x 10⁶ I. E./g Zubereitung.

Die erfindungsgemäßen pharmazeutischen Zubereitungen enthalten den aktiven Bestandteil in Verbindung mit einem dafür pharmazeutisch annehmbaren Trägerstoff und gegebenenfalls weiteren therapeutisch aktiven Bestandteilen. Der Trägerstoff muß annehmbar in dem Sinne sein, daß er mit den anderen Bestandteilen der Formulierung kompatibel ist und keine nachteilige Wirkung auf den Empfänger der Formulierung besitzt.

Die Formulierungen liegen geeigneterweise in Form einer ophthalmologischen, subkutanen, intrakutanen, intramuskulären, intravenösen, intratekalen, intraartikulären, intratumoralen/peritumoralen, intralesionalen/perilesionalen oder topischen Verabreichungsform vor.

Die erfindungsgemäßen Formulierungen können durch jedes der auf dem Gebiet der pharmazeutischen Technologie bekannten Verfahren hergestellt werden. Im wesentlichen enthalten alle Verfahren den Schritt des Zusammenbringens des aktiven Bestandteils mit dem Trägerstoff, gegebenenfalls mit einem oder mehreren zusätzlichen Bestandteil. Im allgemeinen werden die Formulierungen durch gleichmäßiges und inniges Vermischen des aktiven Bestandteiis mit einem flüssigen Träger oder einem feinverteilten festen Träger oder beiden und anschließend, falls erforderlich, Formen des Produktes in die gewünschte Zubereitungsform, hergestellt.

Die erfindungsgemäßen Formulierungen können in Form von diskreten Einheiten vorliegen, wobei jede Form eine bestimmte Menge des aktiven Bestandteils enthält. Sie können ebenfalls in Form eines Pulvers oder in Form eines Granulats oder in Form einer Lösung oder in Form einer Suspension in einer wäßrigen oder nichtwäßrigen Flüssigkeit oder in Form einer Öl-in-Wasser-Emulsion oder Wasser-in-Öl-Emulsion vorliegen. Der aktive Bestandteil kann ebenfalls in Form eines Bolus, eines Elektuariums oder einer Paste vorliegen.

Wenn die erfindungsgemäßen Formulierungen für die parenterale Verabreichung vorgesehen sind, enthalten sie üblicherweise eine sterile wäßrige Zubereitung des aktiven Bestandteils, die vorzugsweise mit dem Blut des Empfängers isotonisch ist. Die erfindungsgemäßen Formulierungen für die parenterale Verabreichung können auch in Form eines Pulvers in einem sterilen Fläschchen, z. B. als Lyophilisat, vorliegen, wobei der aktive Bestandteil unmittelbar vor Gebrauch durch Lösen mit Aqua inj. für die Verabreichung zubereitet wird.

Erfindungsgemäße Zubereitungen, die für die intraartikuläre Verabreichung geeignet sind, können in Form einer sterilen wäßrigen Zubereitung des aktiven Bestandteils vorliegen, wobei der aktive Bestandteil gegebenenfalls in mikrokristalliner Form vorliegt, z. B. in Form einer wäßrigen mikrokristallinen Suspension.

Die erfindungsgemäßen Formulierungen können ebenfalls in Form einer liposomalen Zubereitung oder in Form eines bioabbaubaren Polymersystems zur Verabreichung des aktiven Bestandteils vorliegen.

Für die topische Verabreichung geeignete erfindungsgemäße Formulierungen enthalten flüssige oder halbflüssige Zubereitungen, wie z. B. Einreibemittel, Lotionen, Verbände, Öl-in-Wasser- oder Wasser-in-Öl-Emulsionen, wie z. B. Cremes, Salben oder Pasten, oder Lösungen oder Suspensionen, wie z. B. Tropfen. Zum Beispiel kann der aktive Bestandteil für die ophthalmologische Verabreichung in Form von wäßrigen Augentropen, beispielsweise in Form einer 0,1 bis 1,0 %igen Lösung, vorliegen.

Zusätzlich zu den vorhin genannten Bestandteilen können die erfindungsgemäßen Formulierungen einen oder mehrere weitere übliche und bekannte Komponenten, wie z. B. Verdünnungsmittel, Puffersubstanzen, Bindemittel, oberflächenaktive Mittel, Verdickungsmittel, Gieitmittel, Konservierungsmittel, Antioxidantien, Emulgatoren und dergleichen enthalten.

Das hier verwendete IFN-beta wurde mit dem in der EP-A 287 075 beschriebenen Verfahren produziert, auf die hier ausdrücklich Bezug genommen wird.

Das erfindungsgemäße Verfahren zur Herstellung des reinen rekombinanten IFN-beta liefert sehr reproduzierbar IFN-beta mit einer Kohlenhydratzusammensetzung, die die vorgenannten Merkmale erfüllt. Die Schwankungsbreite ist sehr gering. Das Verfahren führt ebenfalls zu einem hochreinen IFN-beta, das aufgrund seiner hohen Volumenaktivität von 12 bis 50 x 10⁶ IU/ml im Endprodukt die Voraussetzungen für eine high dosage-Formulierung erfüllt.

Dieses hochkonzentrierte rekombinante IFN-beta zeigt im Gegensatz zu IFN-beta geringerer Reinheit und niedrigerer Volumenaktivität eine signifikant erhöhte Eigenstabilität. Diese hohe Stabilität wird ohne Zusätze und Stabilisatoren, wie sie nach dem Stand der Technik erforderlich sind, in gepufferter physiologischer Salzlösung bei neutralem pH-Wert erreicht.

Das in den erfindungsgemäßen Zubereitungen verwendete rekombinante IFN-beta besitzt eine spezifische Aktivität von mindestens 2 x 10⁸ IU/mg. Die Reinheit des verwendeten IFN-beta beträgt mindestens > 98 %, bevorzugt > 99 %.

Die spezifische Aktivität wird dadurch bestimmt, daß man die antivirale Aktivität mit einem NIH-Referenzstandard vergleicht. Die Proteinkonzentration wird mit der Standardmethode nach Lowry bestimmt.

Die folgenden Beispiele dienen der Erläuterung der Erfindung, ohne diese jedoch darauf zu beschränken.

### Beispiel 1

### Herstellung des rekombinanten IFN-beta

Zum Aufbau einer Zellkulturpyramide mit CHO BIC 8622 Zellen werden die Zellen, ausgehend von einer Ampulle, schrittweise in größere Produktionsgefäße überführt und bis zu einem für die rekombinante IFN-beta Produktion geeigneten Maßstab vermehrt. Für den Aufbau der Produktionspyramide werden Roux-Flaschen, Doppelwannen, Wannenstapel und Batteriewannenstapel verwendet. Die Erntephase erfolgt im Batteriewannenstapel. Das Medium besteht aus MEM alpha minus, L-Glutamin (2 mM) und Gentamycin (50 µg/l). Um höhere Ausbeuten zu erzielen, kann 1 % bis 5 % fötales Kälberserum zugesetzt werden. Das rekombinate IFN-beta wird in das Wachstumgsmedium sezerniert. Die Ernte, 80 l konditioniertes Medium mit 1 x 10⁵ bis 1 x 10⁶ IU/l rekombinatem IFN-beta, erfolgt alle 24 Stunden.

Das rekombinante IFN-beta weist in dieser Stufe eine spezifische Aktivität von 1,0 x 10⁶ IU/mg Protein auf.

Zur Phasenverteilung werden 80 l konditioniertes Medium mit 80 l einer Mischung, enthaltend 60 l bidest. Wasser, 8.000 g NaH₂PO₄ x 2 H₂O, 30.000 g K₂HPO₄, 13.000 g NaCl, 9.000 g Polyethylenglykol PEG 2000, in einem Edelstahlkessel gemischt, wobei die Mischungstemperatur 15 °C beträgt. Nach einer Trennzeit von 12 Stunden wird die Unterphase entfernt und die Oberphase, ca. 20 l, für die weitere Reinigung erhalten. Die Ausbeute an rekombinantem IFN-beta beträgt nahezu 100 %.

Das rekombinante IFN-beta weist in dieser Stufe eine spezifische Aktivität von 3 x 10⁶ IU/mg Protein auf.

Die darauffolgenden chromatographischen Reinigungsstufen können aufgrund der hohen Eigenstabilität des rekombinanten IFN-beta bei 15 °C durchgeführt werden.

Das chromatographische Reinigungsverfahren beginnt mit der Affinitätschromatographie an Blue Sepharose FF (Pharmacia). Dazu werden 0,5 l Blue Sepharose FF in eine Chromatographiesäure mit einem Durchmesser von 10 cm gepackt und mit 1,5 l PBS äquilibriert. Die Geibetthöhe beträgt ca. 6 cm. Die Oberphase, 20 l, wird mit PBS, enthaltend 1 mol/l NaCl, 1 : 1 verdünnt und mit einer Flußrate von 3 cm/min über die Säule gepumpt. IFN-beta bindet unter diesen Bedingungen zu 95 %.

Es erfolgt eine Waschung mit 2,0 l PBS, enthaltend 1 mol/l NaCl. Anschließend erfolgt die Elution mit einem Gradienten aus 1,0 l PBS und 1,0 l PBS, enthaltend 30 % w/w Propylenglykol und 10 % w/w Ethylenglykol. Die Flußraten für Waschung und Elution betragen 4 cm/min. In der Waschung werden ca. 10 bis 15 % der Interferonaktivität, in der Elution ca. 80 % der Interferonaktivität erhalten. Das rekombinante IFN-beta weist in dieser Stufe eine spezifische Aktivität von 130 x 10⁶ IU/mg Protein auf. Daran schließt sich die Metallchelatchromatographie an.

Dazu werden 0,25 l Chelating Sepharose (Pharmacia) in eine Chromatographiesäule mit einem Durchmesser von 8 cm gepackt. Die Gelbetthöhe beträgt ca. 4 cm. Die Säule wird mit 1,0 l, 20 mmol/l ZnCl₂-Lösung beladen und anschließend mit 3,0 l aqua bidest. gewaschen und mit 3,0 l PBS, enthaltend 1 mol/l NaCl äquilibriert. Die Flußrate beträgt jeweils 1,5 cm/min.

Das Eluat der Affinitätschromatographie wird mit PBS 1 : 2 verdünnt und mit einer Flußrate von 1,5 cm/min über die Säule gepumpt. IFN-beta bindet unter diesen Bedingungen zu ca. 100 %. Es folgt eine Waschung mit 1,0 l PBS, enthaltend 1 mol/l NaCl und anschließend erfolgt die Elution mit einem Gradienten aus 0,5 l PBS, enthaltend 1 mol/l NaCl und 0,5 l PBS, enthaltend 1 mol/l NaCl und 0,1 mol/l Imidazol. In der Waschung werden ca. 15 bis 20 % der Interferonaktivität, in der Elution ca. 70 % der Interferonaktivität erhalten. Das rekombinante IFN-beta weist in dieser Stufe eine spezifische Aktivität von 195 x 10⁶ IU/mg Protein auf.

Darauf folgt die Size-Exclusion-Chromatographie. Dazu werden 3,0 l Sephacryl ^{R} S-200 (Pharmacia) in eine Chromatographiesäule mit einem Durchmesser von 6 cm gepackt. Die Gelbetthöhe beträgt ca. 90 cm. Die Säule wird mit 6,0 l PBS, enthaltend 0,1 mol/l NaCl, äquilibriert. Die Flußrate beträgt 0,5 cm/min. Der Auftrag auf die SEC-Säule beträgt ca. 0,2 l des Eluats der Metallchelatsäule. Die Flußrate beträgt 0,8 cm/min. Die Chromatographie erfolgt mit 3,0 l PBS, enthaltend 0,1 mol/l NaCl, mit einer Flußrate von 0,9 cm/min. In der Elution werden ca. 70 % der Interferonaktivität erhalten. Das rekombinante IFN-beta weist eine spezifische Aktivität von 220 x 10⁶ IU/mg und eine Proteinreinheit, bestimmt durch SDS-PAGE Elektrophorese und Silver staining, von > 99 % auf.

Das Eluat der Size-Exclusion-Chromatographie wird auf Identität (Primärsequenz, Kohlenhydratstrukturen) und Wirksamkeit (Biologischer Assay, ELISA) getestet und anschließend formuliert.

### Beispiel 2

### Formulierung des rekombinanten IFN-beta

Zur Formulierung wird das Eluat der Size-Exclusion-Chromatographie, ca. 0,8 l mit einer Interferonaktivität von 40 x 10⁶ IU/ml und einem Proteingehalt von 0,18 mg/ml, mit PBS auf die gewünschte Volumenaktivität von z. B. 30 x 10⁶ IU/ml verdünnt und mit 10 mg/ml humanen Serumalbumin als Füllstoff versetzt. Diese Lösung wird durch ein 0,22 µm Filter steril filtriert. Von dieser Formulierung werden 1 ml Aliquots in DIN R2 Flaschen abgefüllt und bei - 50 °C tiefgefroren und anschließend lyophilisiert. Die verschlossenen gefriergetrockneten Fläschchen werden bei 15 °C gelagert.

### Beispiel 3

Beispiel 3 zeigt die Stabilität von Zubereitungen mit rekombinantem IFN-beta aus CHO-Zellen mit einer Reinheit von > 99 % in flüssiger Formulierung in unterschiedlicher Zusammensetzung ohne stabilisierende Zusätze.

Gelöstes rekombinantes IFN-beta mit 10⁸ IU/ml und einem Proteingehalt von 0,4 mg/ml wird gegen die beschriebenen Formullerungspuffer dialysiert und anschließend mit dem entsprechenden Puffer auf die geforderte Volumenaktivität verdünnt.

Die Lösung wird dann durch ein 0,22 µm Filter steril filtriert. Anschließend wird von dieser Formulierung je 1 ml in sterilisierte Glasflaschen DIN 3 R (3 ml Volumen) abgefüllt und mit einem Stopfen verschlossen. Von jeder Formulierung werden eine geeignete Anzahl von Fläschchen hergestellt, so daß für jede Stabilitätsprüfung eine neue Flasche zur Verfügung steht.

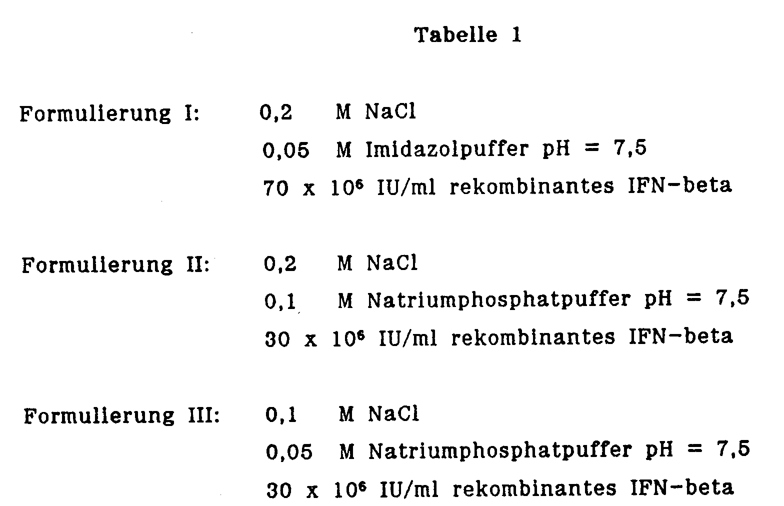

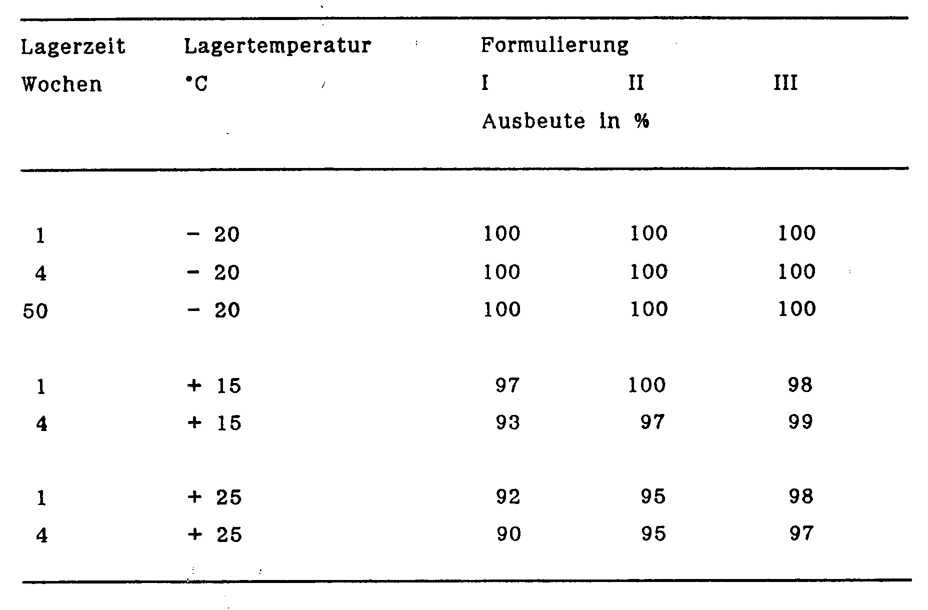

Tabelle 1 zeigt, daß die hohe Stabilität der erfindungsgemäßen Zubereitungen nicht von der Zusammensetzung des Puffers abhängt.

### Beispiel 4

Beispiel 4 zeigt die Stabilität von Zubereitungen mit rekombinantem IFN-beta aus CHO-Zellen mit einer Reinheit von > 99 % in lyophillsierter Form unterschiedlicher Zusammensetzung mit humanem Serumalbumin als Füllstoff. Es wurden keine stabilisierenden Zusätze verwendet.

Gelöstes rekombinantes IFN-beta mit einer Aktivität von 1,5 x 10⁸ IU/ml und einem Proteingehalt von 0,6 mg/ml wird gegen die angegebenen Formulierungspuffer ohne HSA dialysiert und anschließend mit dem entsprechenden Puffer mit HSA auf die geforderte Volumenaktivität verdünnt.

Die Lösung wird dann durch ein 0,22 µm Filter sterilfiltriert. Anschließend wird von jeder Formulierung je 1 ml in sterilisierte Glasflaschen DIN 3 R (3 ml Volumen) abgefüllt und lyophilisiert.

Von jeder Formulierung werden eine geeignete Anzahl von Fläschchen hergestellt, so daß für jede Stabilitätsprüfung eine neue Flasche zur Verfügung steht. Nach Beendigung des Belastungsexperiments wird der Inhalt einer Flasche in 1 ml Aqua inj. gelöst und der verbleibende Wirkstoffgehalt mittels Bioassay bestimmt.

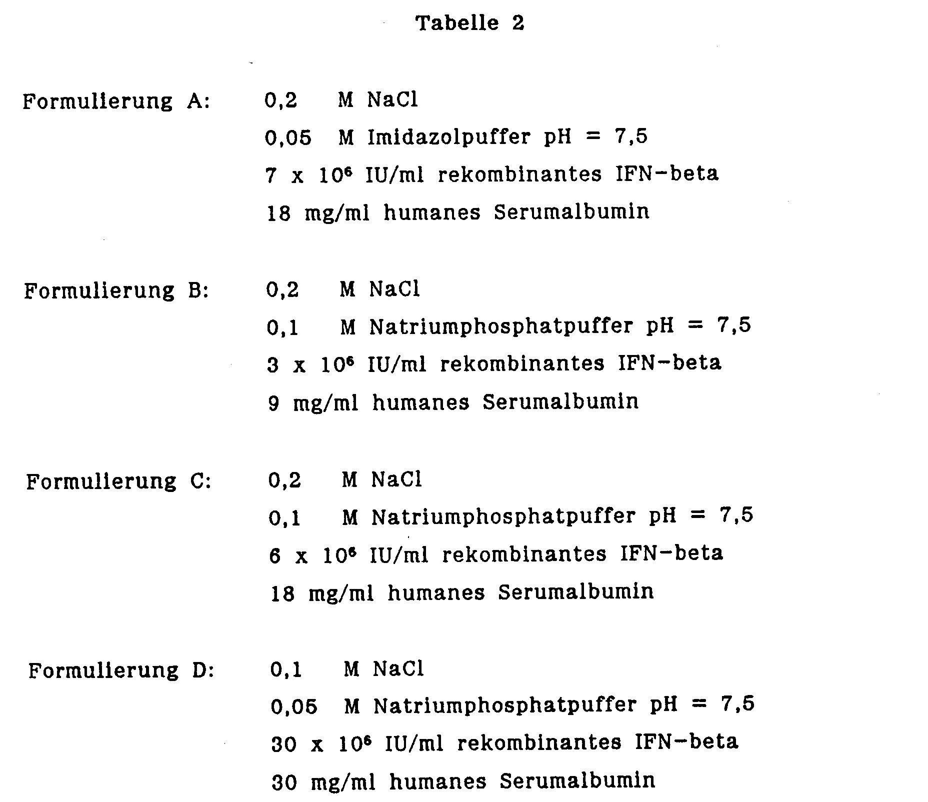

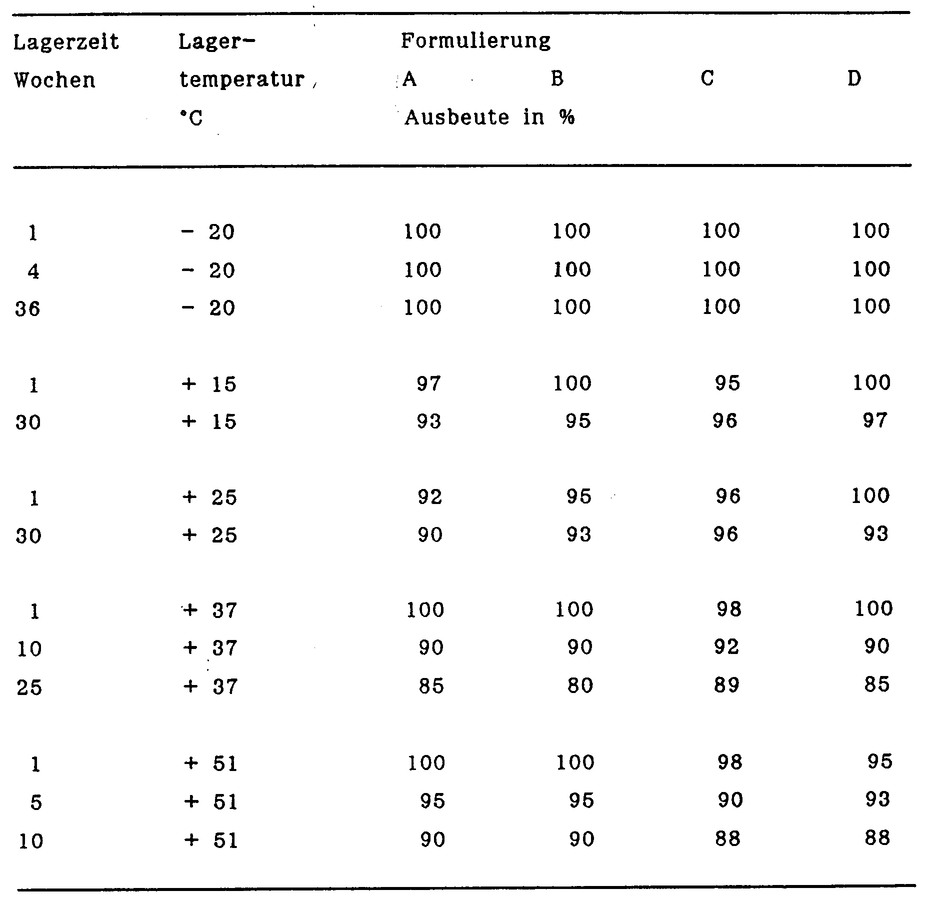

Die Ergebnisse der Tabelle 2 zeigen, daß die erfindungsgemäßen Formulierungen mit rekombinantem IFN-beta eine stabile Endformullerung in physiologischem Puffer bei neutralem pH-Wert ohne weitere Zusätze zur Stabilisierung ermöglichen. Das als Zusatz verwendete Serumalbumin dient als Füllstoff für das lyophilisierte Protein, da der Proteingehalt des reinen IFN-beta nur 10 bis 200 µg beträgt.

Die Ergebnisse zeigen weiterhin, daß die Formulierungen auch in lyophillsierter Form sehr stabil sind. Der Vorteil dieser stabilen Formulierung des Wirkstoffes liegt in der langen Haltbarkeit. Ein weiterer Vorteil ist in der Lagertemperatur bei + 4 bis + 25 °C zu sehen, da eine Kühlkette, wie normalerweise für Interferonzubereitungen erforderlich, entfällt.

### Beispiel 5

Dieses Beispiel zeigt die Stabilität von Zubereitungen mit rekombinantem IFN-beta aus CHO-Zellen mit einer Reinheit von > 99 % in lyophilisierter Form unterschiedlicher Zusammensetzung und mit humanem Serumalbumin als Füllstoff nach Rekonstitution in 1 ml Aqua inj..

Gelöstes rekombinantes IFN-beta mit einer Aktivität von 1,5 x 10⁸ IU/ml und einem Proteingehalt von 0,6 mg/ml wird gegen die angegebenen Formulierungspuffer ohne HSA dialysiert und anschließend mit dem entsprechenden Puffer mit HSA auf die geforderte Volumenaktivität verdünnt.

Die Lösung wird dann durch ein 0,22 µm Filter sterilfiltriert. Anschließend wird von jeder Formulierung je 1 ml in sterilisierte Glasflaschen DIN 3 R (3 ml Volumen) abgefüllt, mit einem Stopfen versehen und lyophillsiert.

Von jeder Formulierung werden eine geeignete Anzahl von Fläschchen hergestellt, so daß für jede Stabilitätsprüfung eine neue Flasche zur Verfügung steht. Nach einer Lagerzeit von 4 Wochen bei + 4 °C wird das Lyophilisat in 1 ml Aqua inj. gelöst. Diese Lösung wird dann für die Stabilitätsuntersuchungen verwendet.

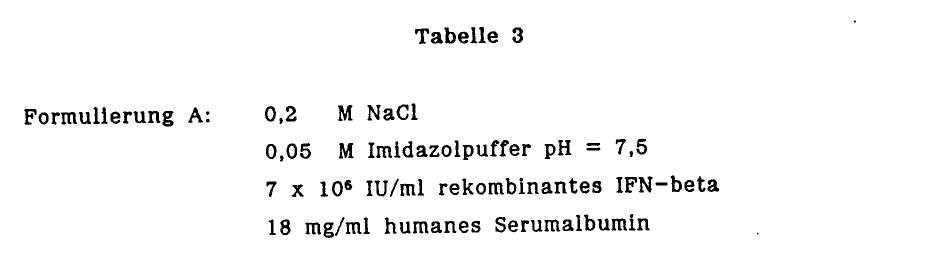

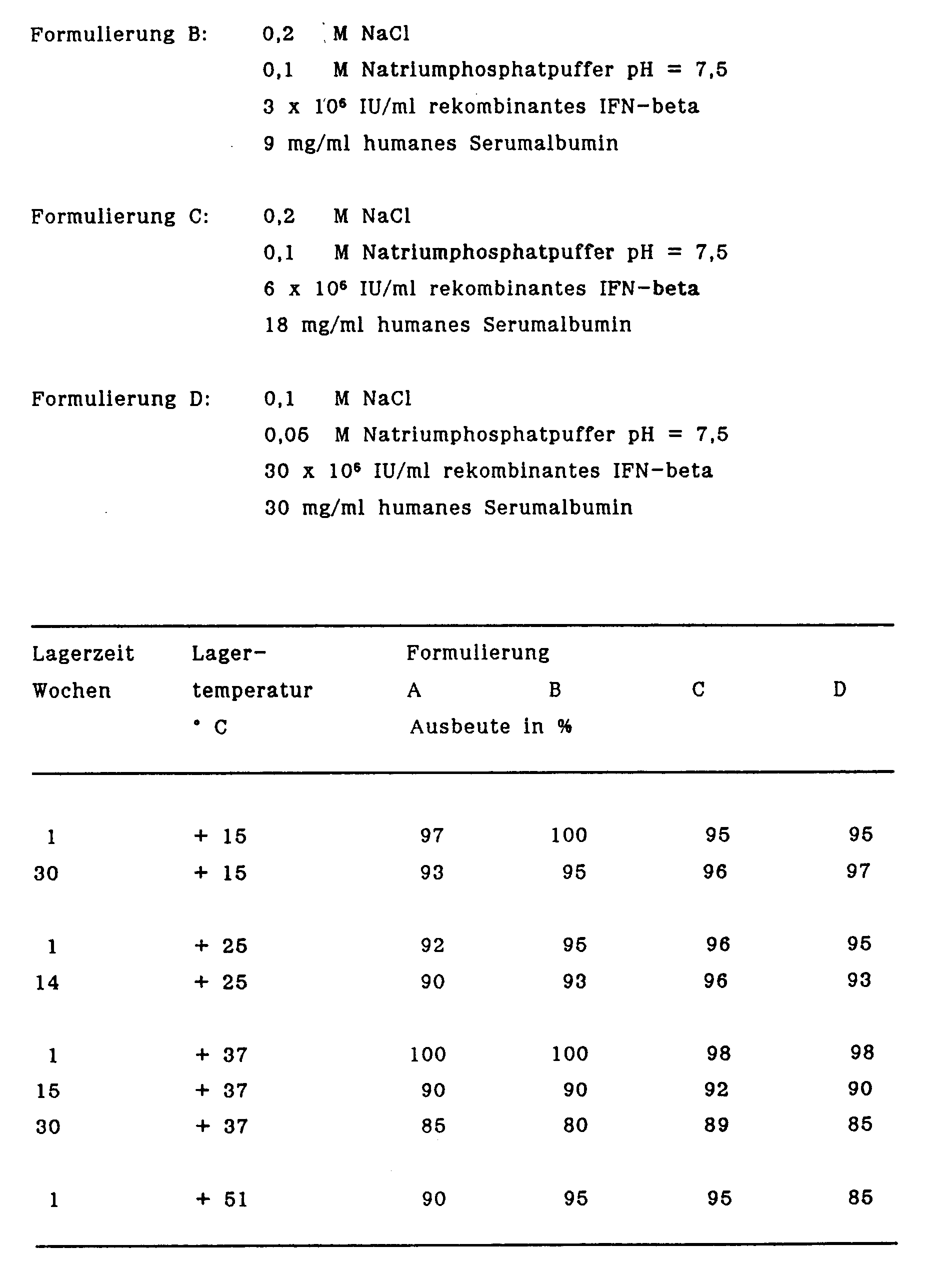

Die hohe Stabilität der erfindungsgemäßen Zubereitungen wird, wie die Tabelle 3 zeigt, nicht von der Zusammensetzung des Puffers beeinflußt. Dies zeigt, daß die erfindungsgemäßen Zubereitungen nach Lyophilisation und Rekonstitution sehr stabil sind. Der Vorteil dieser stabilen Formulierung des Wirkstoffs zeigt sich vorteilhafterweise vor allem bei Infusionen über einen längeren Zeitraum bei Raumtemperatur.

### Beispiel 6

Dieses Beispiel zeigt die reduzierte Stabilität von Zubereitungen mit rekombinantem IFN-beta aus CHO-Zellen mit einer Reinheit von > 99 % nach Behandlung mit Sialidase. Durch dieses Enzym werden definiert endständige Sialinsäuren enzymatisch abgespalten.

1 mg rekombinantes IFN-beta mit einer Reinheit von > 99 % wird in 0,05 mol/l Natriumacetatpuffer, pH = 5,5, mit 0,02 mg Neuraminidase (Sialidase), Boehringer Mannheim, versetzt und bei 25 °C 2 bis 6 Stunden inkubiert. Die freigesetzte Sialinsäure wird über Anionenaustauschchromatographie bestimmt. Bei einem verbleibenden Sialinsäuregehalt von ca. 60 bis 70 % wird eine Ultrafiltration mit einer Ausschlußgrenze von 30.000 D durchgeführt. Die Neuraminidase wird im Konzentrat erhalten, während das rekombinante IFN-beta im Permeat vorliegt. Nach Dialyse gegen Formulierungspuffer II und III werden die Stabilitäten wie in Beispiel 1 beschrieben bestimmt.

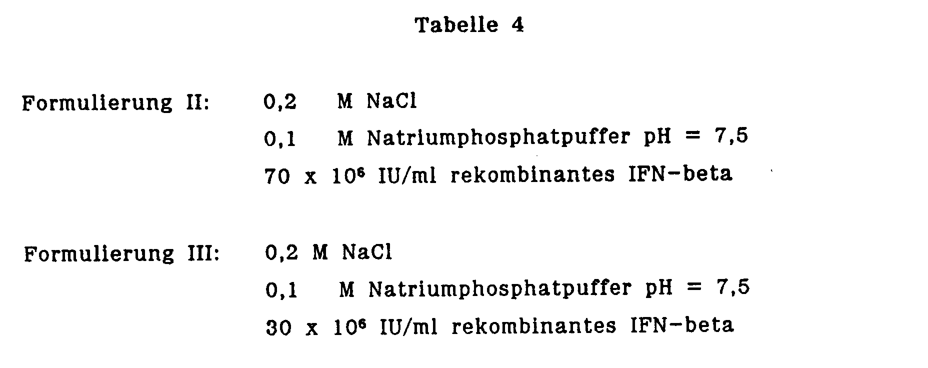

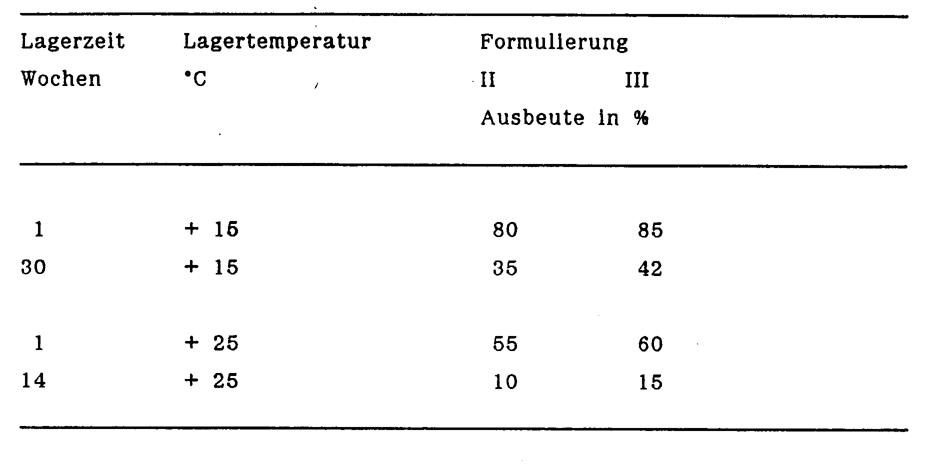

Beispiel 6 zeigt den Zusammenhang zwischen Sialisierungsgrad und Stabilität von rekombinantem IFN-beta.

Der deutlich geringere Sialinsäuregehalt von ca. 60 bis 70 % führt zu einer erhöhten Instabilität des IFN-beta. Mit der Reduzierung des Sialinsäuregehaltes geht parallel eine Reduzierung der Löslichkeit einher und das IFN-beta neigt zu Trübungen und Präzipitationen.

### Beispiel 7

In diesem Beispiel wurde die Zusammensetzung des Kohlenhydratanteils von IFN-beta, produziert in FS4-Zellen in Zellkultur, und dem erfindungsgemäßen rekombinantem IFN-beta, produziert in CHO-Zellkulturen, untersucht. Soweit noch nicht angegeben, sind die Verfahren zur Bestimmung des Kohlenhydratanteils in The Journal of Biological Chemistry, Vol. 262, Nr. 30, Oktober 25, 1987, Seite 25, beschrieben. Die Primärstrukturen des rekombinanten IFN-beta aus CHO-Zellen und des IFN-beta aus FS4-Zellen sind identisch.

Im Vergleich zum natürlichen IFN-beta aus Fibroblasten (FS4) zeigt das erfindungsgemäße rekombinante IFN-beta aus CHO-Zellen einen deutlich erhöhten Anteil im Bereich der triantennären und höhersialisierten Strukturen. Der Sialylierungsgrad bei natürlichem IFN-beta liegt bei ca. 70 %, beim erfindungsgemäßen rekombinantem IFN-beta über 90 %.

Das wie vorstehend beschrieben hergestellte rekombinante IFN-beta ist überraschend klinisch wesentlich wirksamer als natürliches IFN-beta, wie die folgenden Beispiele zeigen.

### Beispiel 8

### Klinische Versuche

- Patient:: männlich, 43 Jahre, homosexuell
- Erkrankung:: AIDS, Klassifikation: CDC IV/WR 5 Kaposi-Sarkome am gesamten Integument mit Beteiligung der Mundschleimhaut
- Therapie:: 3 x 10⁶ IU erfindungsgemäßes rekombinantes IFN-beta, wie o. a., subcutan 3 x/Woche über 9 Wochen plus Zidovudine 500 mg/d
- Ergebnis:: Verringerung der Kaposi-Sarkome in Größe und Anzahl
- Therapie:: 3 x 10⁶ IU hochgereinigtes natürliches IFN-beta subcutan 3 x/Woche über 6 Wochen plus Zidovudine 500 mg/d
- Ergebnis:: Auftreten neuer Kaposi-Sarkome
- Therapie:: 3 x 10⁶ IU erfindungsgemäßes rekombinantes IFN-beta, wie o. a., subcutan 3 x/Woche kontinuierlich plus Zidovudine 500 mg/d über ca. 6 Wochen
- Ergebnis:: Verringerung der Kaposi-Sarkome in Größe und Anzahl

### Beispiel 7

### Klinische Versuche

- Patient:: männlich, 47 Jahre, homosexuell
- Erkrankung:: AIDS, Klassifikation: CDC IV/WR 5 Kaposi-Sarkome am gesamten Integument
- Therapie:: 3 x 10⁶ IU erfindungsgemäßes rekombinantes IFN-beta, wie o. a., subcutan 3 x/Woche über 5 Wochen plus Zidovudine 1000 mg/d
- Ergebnis:: Verringerung der Kaposi-Sarkome in Größe und Anzahl
- Therapie:: 3 x 10⁶ IU hochgereinigtes natürliches IFN-beta subcutan 3 x/Woche über 6 Wochen plus Zidovudine 500 mg/d
- Ergebnis:: Vergrößerung der Kaposi-Sarkome
- Therapie:: 3 x 10⁶ IU erfindungsgemäßes rekombinantes IFN-beta, wie o. a., subcutan 3 x/Woche kontinuierlich plus Zidovudine 500 mg/d über ca. 6 Wochen
- Ergebnis:: Verringerung der Kaposi-Sarkome in Größe und Anzahl

## Patentansprüche (Patentansprüche für folgende(n) Vertragsstaat(en): BE, CH, LI, DE, DK, FR, GB, IT, LU, MC, NL, PT, SE)

1. Rekombinantes Human IFN-beta aus CHO-Zellkulturen, gekennzeichnet dadurch,
daß es einen Anteil an biantennären Oligosaccharid-Strukturen von mindestens 70 %, einen Anteil von triantennären Oligosaccharid-Strukturen von mindestens 20 %, und einen Anteil von tetraantennären Oligosaccharid-Strukturen von 0 bis 5 % sowie einen Sialinsäuregehalt von mindestens 90 % aufweist.

2. Rekombinantes Human-IFN-beta nach Anspruch 1, dadurch gekennzeichnet, daß der Anteil an blantennären Oligosaccharid-Strukturen mindestens 75 % beträgt.

3. Rekombinantes Human-IFN-beta nach Anspruch 1 oder 2, dadurch gekennzeichnet, daß der Anteil an triantennären Oligosaccharid-Strukturen mindestens 25 % beträgt.

4. Rekombinantes Human-IFN-beta nach einem der Ansprüche 1 bis 3, dadurch gekennzeichnet, daß die triantennären Strukturen gegebenenfalls mindestens ein N-Acetyllactosamin-Repeat aufweisen.

5. Rekombinantes Human-IFN-beta nach Anspruch 1 bis 4, dadurch gekennzeichnet, daß die triantennäre Struktur 1->4 und/oder 1->6 verknüpft ist.

6. Rekombinantes Human-IFN-beta nach einem der Ansprüche 1 bis 5, dadurch gekennzeichnet, daß der Anteil an tetraantennären Oligosaccharid-Strukturen 0,5 bis 3 % beträgt.

7. Rekombinantes Human-IFN-beta nach einem der Ansprüche 1 bis 6, dadurch gekennzeichnet, daß sich der Sialinsäureanteil aus N-Acetylneuraminsäure und N-Glykolylneuraminsäure zusammensetzt.

8. Rekombinantes Human-IFN-beta nach Anspruch 7, dadurch gekennzeichnet, daß der Anteil an N-Acetylneuraminsäure 90 bis 100 % und der Anteil an N-Glykolylneuraminsäure 0 bis 10 % beträgt.

9. Rekombinantes Human-IFN-beta nach einem der Ansprüche 1 bis 8, dadurch gekennzeichnet, daß das rekombinante IFN-beta weiterhin einen Fucosegehalt von mindestens 85 %, bevorzugt 90 % und am meisten bevorzugt > 95 % aufweist.

10. Rekombinantes Human-IFN-beta nach einem der Ansprüche 1 bis 9, dadurch gekennzeichnet, daß mindestens eine Oligosaccharid-Struktur einer der folgenden Formeln entspricht: 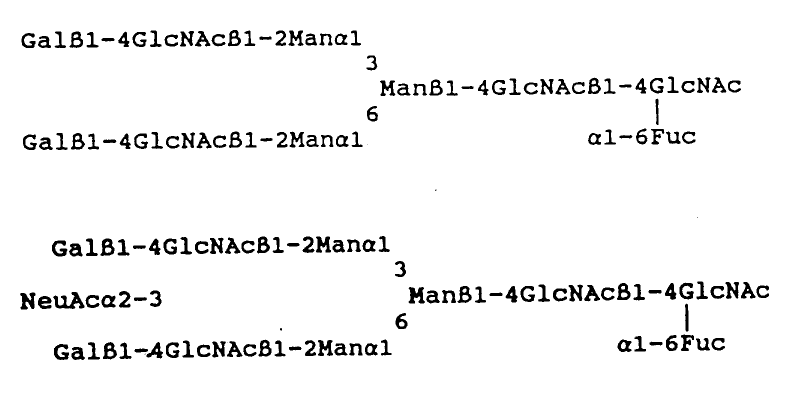 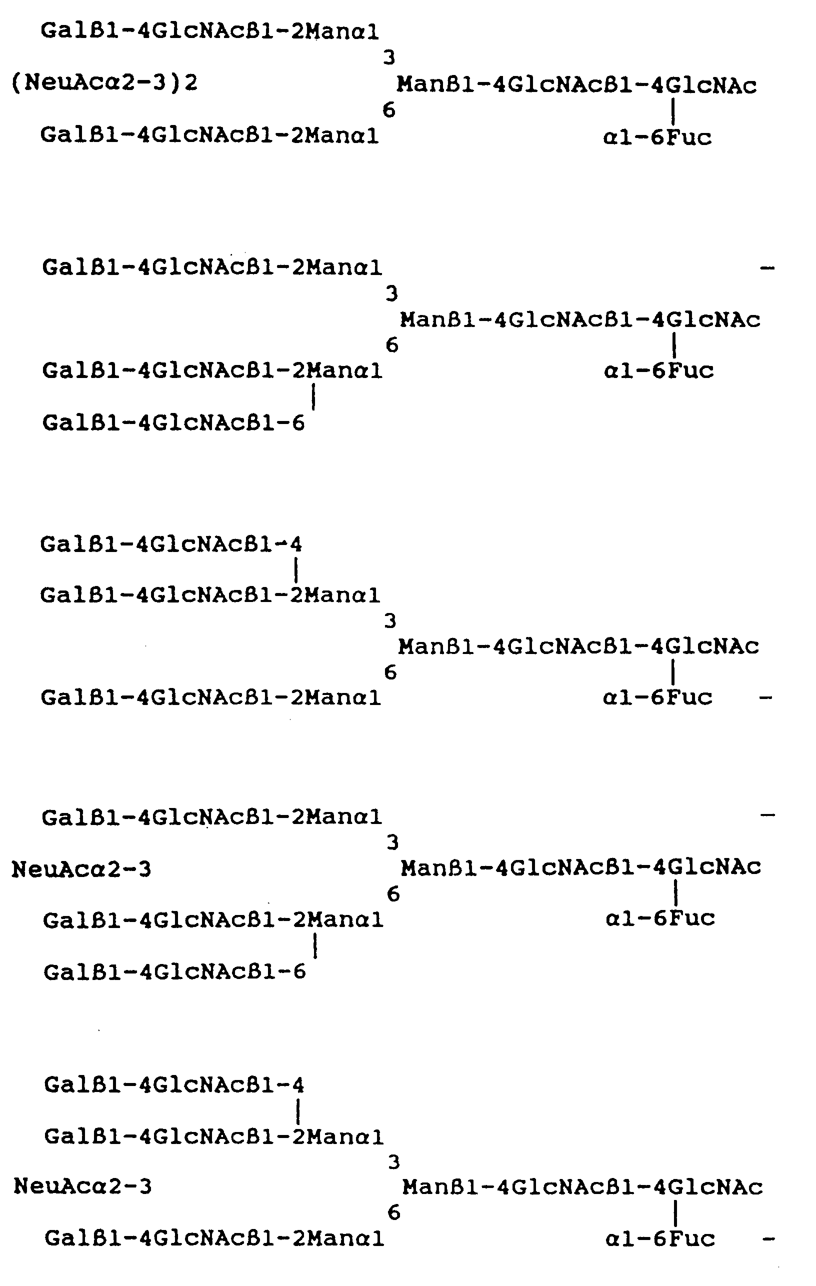 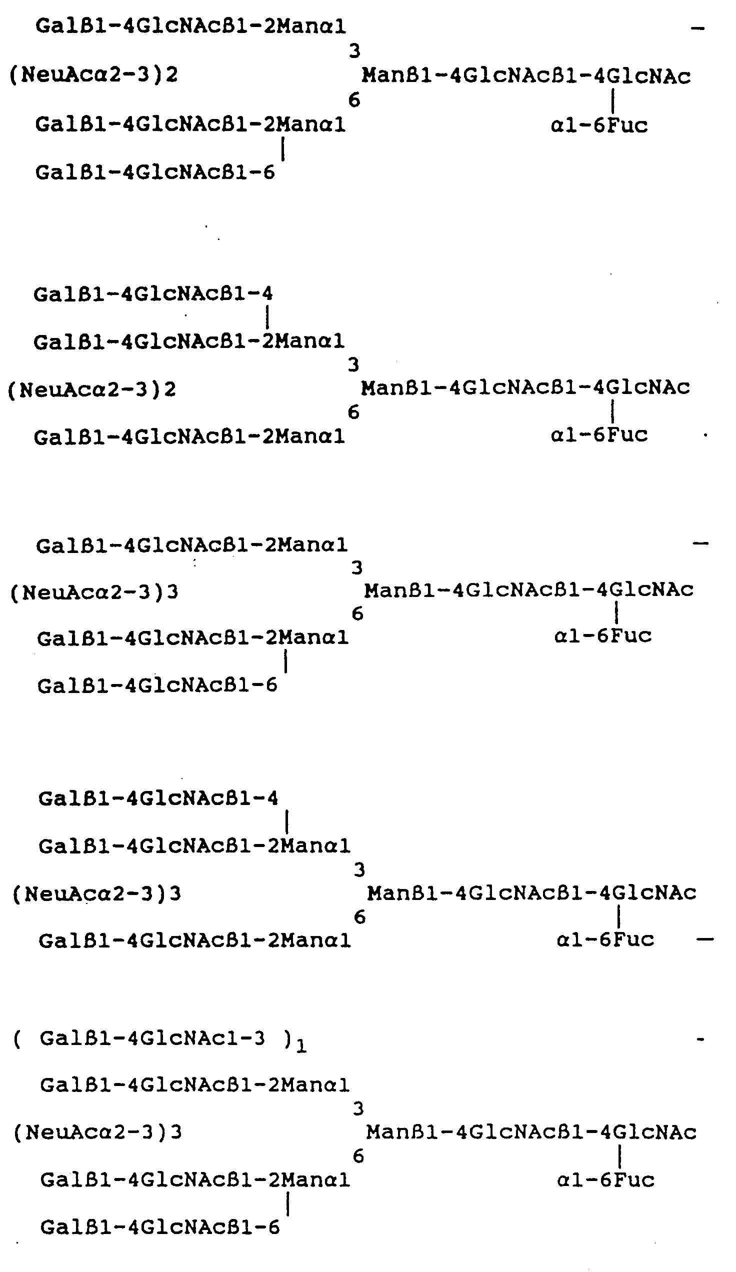 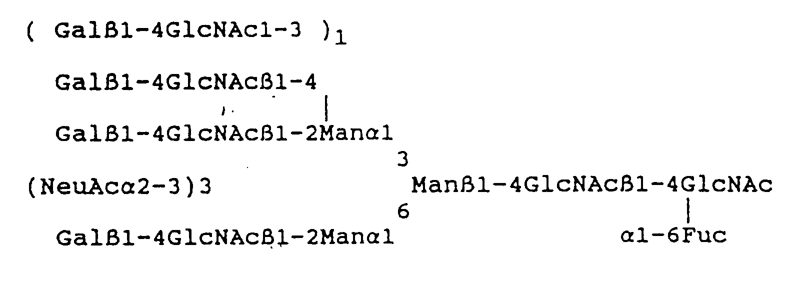 wobei NeuAc auch für N-Glykolylneuraminsäure steht.

11. Rekombinantes Human-IFN-beta nach einem der Ansprüche 1 bis 10, dadurch gekennzeichnet, daß es eine spezifische Aktivität von mindestens 200 x 10⁶ IU/mg aufweist.

12. Verfahren zur Herstellung von rekombinantem Human-IFN-beta nach einem der Ansprüche 1 bis 11, dadurch gekennzeichnet, daß eine wäßrige Lösung von Human-IFN-beta in unreinem Zustand durch eine flüssig/flüssig Phasenextraktion angereichert wird, dann einer Farbstoff-Affinitätschromatographie unterzogen wird, anschließend eine Metallchelatchromatographie durchgeführt wird, der eine Size-Exclusion-Chromatographie folgt, wobei die jeweiligen Chromatographieschritte in diese Reihenfolge durchgeführt werden.

13. Verfahren nach Anspruch 12, dadurch gekennzeichnet, daß die flüssig/flüssig Phasenextraktion mit einem wäßrigen Zweiphasensystem auf Basis Polyalkylenglykol/Dextran oder Polyalkylenglykol/Salz durchgeführt wird.

14. Verfahren nach Anspruch 13, dadurch gekennzeichnet, daß als Polyalkylenglykol Polyethylenglykol mit einem Molekulargewicht von 1.000 bis 6.000 oder Polypropylenglykol mit einem Molekulargewicht von 1.500 bis 4.000 verwendet wird.

15. Verfahren nach Anspruch 13, dadurch gekennzeichnet, daß als Salz NaCl, LiCl, NaJ, KJ, Na₂SO₄, Na₂HPO₄, K₂HPO₄, K₂SO₄, NaH₂PO₄, KCl, NH₄Cl, (NH₄)₂SO₄, Na-Citrat oder Na-Oxalat verwendet wird.

16. Verfahren nach Anspruch 13, dadurch gekennzeichnet, daß der Farbstoff in der Affinitätschromatographie Cibacron Blau F 3 GA ist.

17. Verfahren nach Anspruch 12, dadurch gekennzeichnet, daß in der Metallchelatchromatographie Cu²⁺, Zn²⁺, Co²⁺ oder Ni²⁺ Ionen verwendet werden.

18. Verfahren nach Anspruch 12, dadurch gekennzeichnet, daß für die Size-Exclusion-Chromatographie Trennmedien verwendet werden, die einen Trennbereich von 1.000 Dalton bis 600.000 Dalton, vorzugsweise Sephadex ® G150, Sephadex ^{R} G150 superfine, Sephacryl ® S-200 High Resolution, Superose 12 prep grade oder TSK-SW 3000 verwendet werden.

19. Verfahren nach einem der Ansprüche 13 und 16, dadurch gekennzeichnet, daß die Elution in der Affinitätschromatographie mit PBS, enthaltend 10 bis 70 Gew.-% Ethylenglykol und/oder 20 bis 50 Gew.-% Propylenglykol, erfolgt.

20. Verfahren nach einem der Ansprüche 13 und 17, dadurch gekennzeichnet, daß die Elution in der Metallchelatchromatographie mit kompetetiven Substanzen wie Imidazol, Histidin, Glycin oder NH₄Cl oder einem pH-Gradienten von etwa pH 7 bis etwa pH 2 oder durch isokratische Elution mit fallenden pH-Werten erfolgt.

21. Verfahren nach einem der Ansprüche 13 und 18, dadurch gekennzeichnet, daß die Elution in der Size-Exclusion-Chromatographie mit PBS, enthaltend 0 bis 0,5 mol/l NaCl erfolgt.

22. Pharmazeutische Zubereitung enthaltend rekombinantes Human-IFN-beta nach einem der Ansprüche 1 bis 13 in Verbindung mit üblichen Träger- und Hilfsstoffen.

23. Pharmazeutische Zubereitung nach Anspruch 22, dadurch gekennzeichnet, daß sie mindestens 25 x 10⁶ IU/ml IFN-beta enthält.

24. Pharmazeutische Formulierung nach Anspruch 22 oder 23, dadurch gekennzeichnet, daß sie in einer für die topische oder parenterale Verabreichung geeigneten Form vorliegt.

25. Pharmazeutische Formulierung nach Anspruch 23, dadurch gekennzeichnet, daß sie in Form eines Gels, einer Emulsion oder einer Salbe vorliegt.

26. Pharmazeutische Zubereitung nach Anspruch 23, dadurch gekennzeichnet, daß sie in Form eines Lyophilisates, einer Lösung oder einer Infusion vorliegt.

## Patentansprüche (Patentansprüche für folgende(n) Vertragsstaat(en): AT, ES, GR)

1. Verfahren zur Herstellung von rekombinantem Human-IFN-beta aus CHO-Zellkulturen mit einen Anteil an biantennären Oligosaccharid-Strukturen von mindestens 70 %, einen Anteil von triantennären Oligosaccharid-Strukturen von mindestens 20 %, und einen Anteil von tetraantennären Oligosaccharid-Strukturen von 0 bis 5 % sowie einen Sialinsäuregehalt von mindestens 90 %, dadurch gekennzeichnet, daß eine wäßrige Lösung von Human-IFN-beta in unreinem Zustand durch eine flüssig/flüssig Phasenextraktion angereichert wird, dann einer Farbstoff-Affinitätschromatographie unterzogen wird, anschließend eine Metallchelatchromatographie durchgeführt wird, der eine Size-Exclusion-Chromatographie folgt, wobei die jeweiligen Chromatographieschritte in dieser Reihenfolge durchgeführt werden.

2. Verfahren nach Anspruch 1, dadurch gekennzeichnet, daß die flüssig/flüssig Phasenextraktion mit einem wäßrigen Zweiphasensystem auf Basis Polyalkylenglykol/Dextran oder Polyalkylenglykol/Salz durchgeführt wird.

3. Verfahren nach Anspruch 2, dadurch gekennzeichnet, daß als Polyalkylenglykol Polyethylenglykol mit einem Molekulargewicht von 1.000 bis 6.000 oder Polypropylenglykol mit einem Molekulargewicht von 1.500 bis 4.000 verwendet wird.

4. Verfahren nach Anspruch 2, dadurch gekennzeichnet, daß als Salz NaCl, LiCl, NaJ, KJ, Na₂SO₄, Na₂HPO₄, K₂HPO₄, K₂SO₄, NaH₂PO₄, KCl, NH₄Cl, (NH₄)₂SO₄, Na-Citrat ode Na-Oxalat verwendet wird.

5. Verfahren nach Anspruch 2, dadurch gekennzeichnet, daß der Farbstoff in der Affinitätschromatographie Cibacron Blau F 3 GA ist.

6. Verfahren nach Anspruch 1, dadurch gekennzeichnet, daß in der Metallchelatchromatographie Cu²⁺, Zn²⁺, Co²⁺ oder Ni²⁺ Ionen verwendet werden.

7. Verfahren nach Anspruch 1, dadurch gekennzeichnet, daß für die Size-Exclusion-Chromatographie Trennmedien verwendet werden, die einen Trennbereich von 1.000 Dalton bis 600.000 Dalton, vorzugsweise Sephadex^{(R)}G150, Sephadex^{(R)}G150 superfine, Sephacryl^{(R)}S-200 High Resolution, Superose 12 prepgrade oder TSK-SW 3000 verwendet werden.

8. Verfahren nach einem der Ansprüche 2 und 5, dadurch gekennzeichnet, daß die Elution in der Affinitätschromatographie mit PBS, enthaltend 10 bis 70 Gew.-% Ethylenglykol und/oder 20 bis 50 Gew.-% Propylenglykol, erfolgt.

9. Verfahren nach einem der Ansprüche 2 und 6, dadurch gekennzeichnet, daß die Elution in der Metallchelatchromatographie mit kompetetiven Substanzen wie Imidazol, Histidin, Glycin oder NH₄Cl oder einem pH-Gradienten von etwa pH 7 bis etwa pH 2 oder durch isokratische Elution mit fallenden pH-Werten erfolgt.

10. Verfahren nach einem der Ansprüche 2 und 7, dadurch gekennzeichnet, daß die Elution in der Size-Exclusion-Chromatographie mit PBS, enthaltend 0 bis 0,5 mol/l NaCl erfolgt.

11. Verfahren nach Anspruch 1, dadurch gekennzeichnet, daß der Anteil an biantennären Oligosaccharid-Strukturen mindestens 75 % beträgt.

12. Verfahren nach Anspruch 1 oder 11, dadurch gekennzeichnet, daß der Anteil an triantennären Oligosaccharid-Strukturen mindestens 25 % beträgt.

13. Verfahren nach einem der Ansprüche 1 ode 11 - 12, dadurch gekennzeichnet, daß die triantennären Strukturen gegebenenfalls mindestens ein N-Acetyllactosamin-Repeat aufweisen.

14. Verfahren nach Anspruch 1 oder 11 - 13, dadurch gekennzeichnet, daß die triantennäre Struktur 1 → 4 und/oder 1 → 6 verknüpft ist.

15. Verfahren nach einem der Ansprüche 1 oder 11 - 14, dadurch gekennzeichnet, daß der Anteil an tetraantennären Oligosaccharid-Strukturen 0,5 bis 3 % beträgt.

16. Verfahren nach einem der Ansprüche 1 oder 11 - 15, dadurch gekennzeichnet, daß sich der Sialinsäureanteil aus N-Acetylneuraminsäure und N-Glykolylneuraminsäure zusammensetzt.

17. Verfahren nach Anspruch 16, dadurch gekennzeichnet, daß der Anteil an N-Acetylneuraminsäure 90 bis 100 % und der Anteil an N-Glykolylneuraminsäure 0 bis 10 % beträgt.

18. Verfahren nach einem der Ansprüche 1 oder 11 - 17, dadurch gekennzeichnet, daß das rekombinante IFN-beta weiterhin einen Fucosegehalt von mindestens 85 %, bevorzugt 90 % und am meisten bevorzugt > 95 % aufweist.

19. Verfahren nach einem der Ansprüche 1 oder 11 - 18, dadurch gekennzeichnet, daß mindestens eine Oligosaccharid-Struktur einer der folgenden Formeln entspricht: 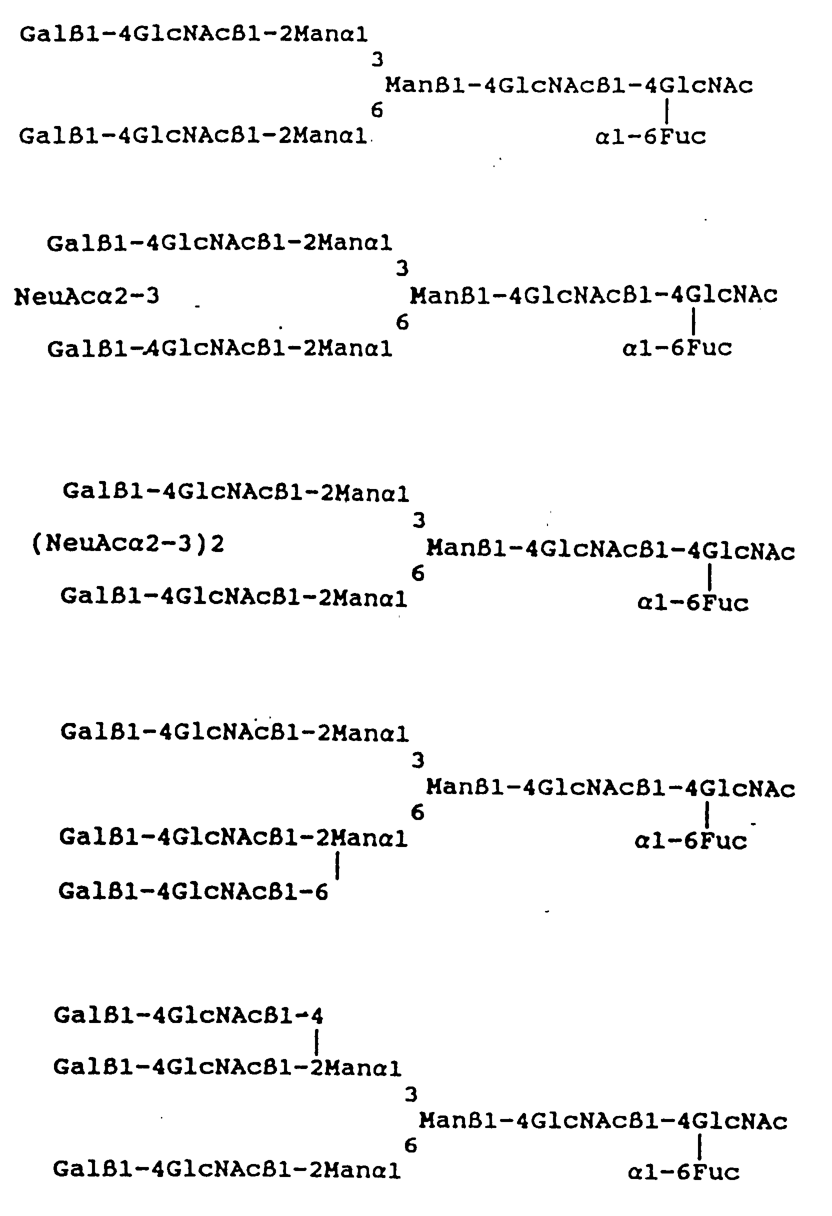 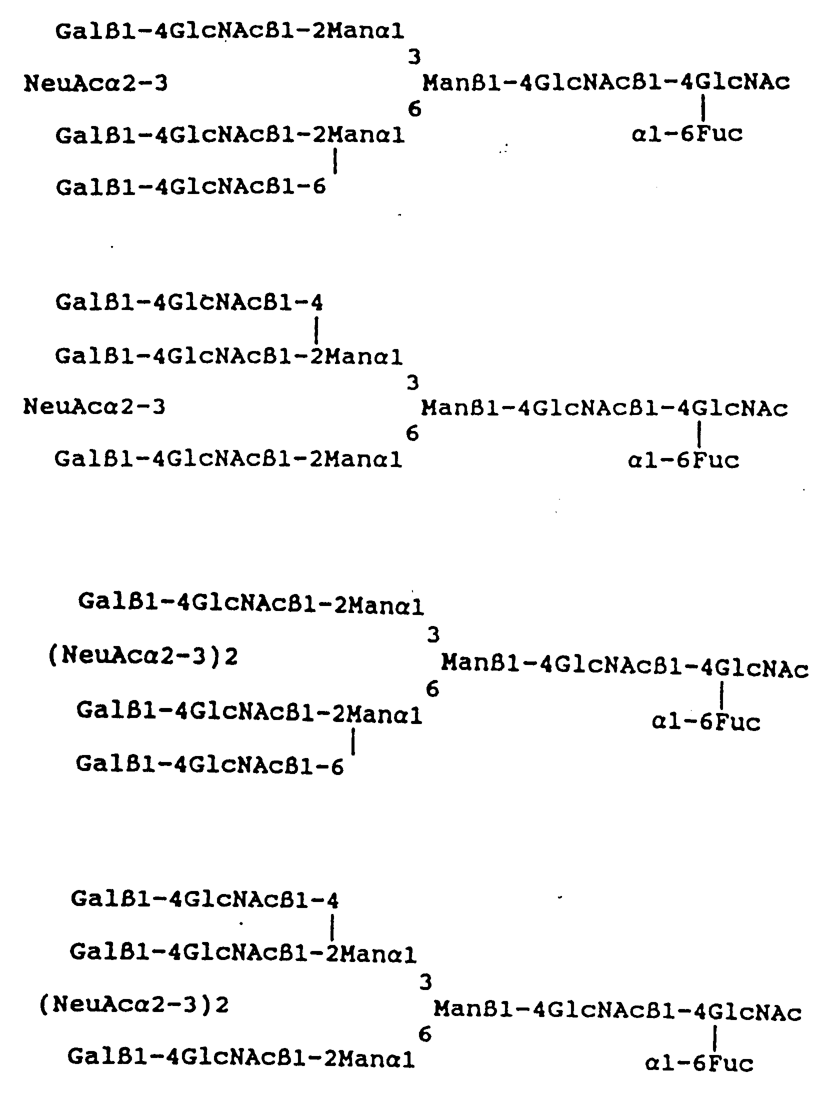 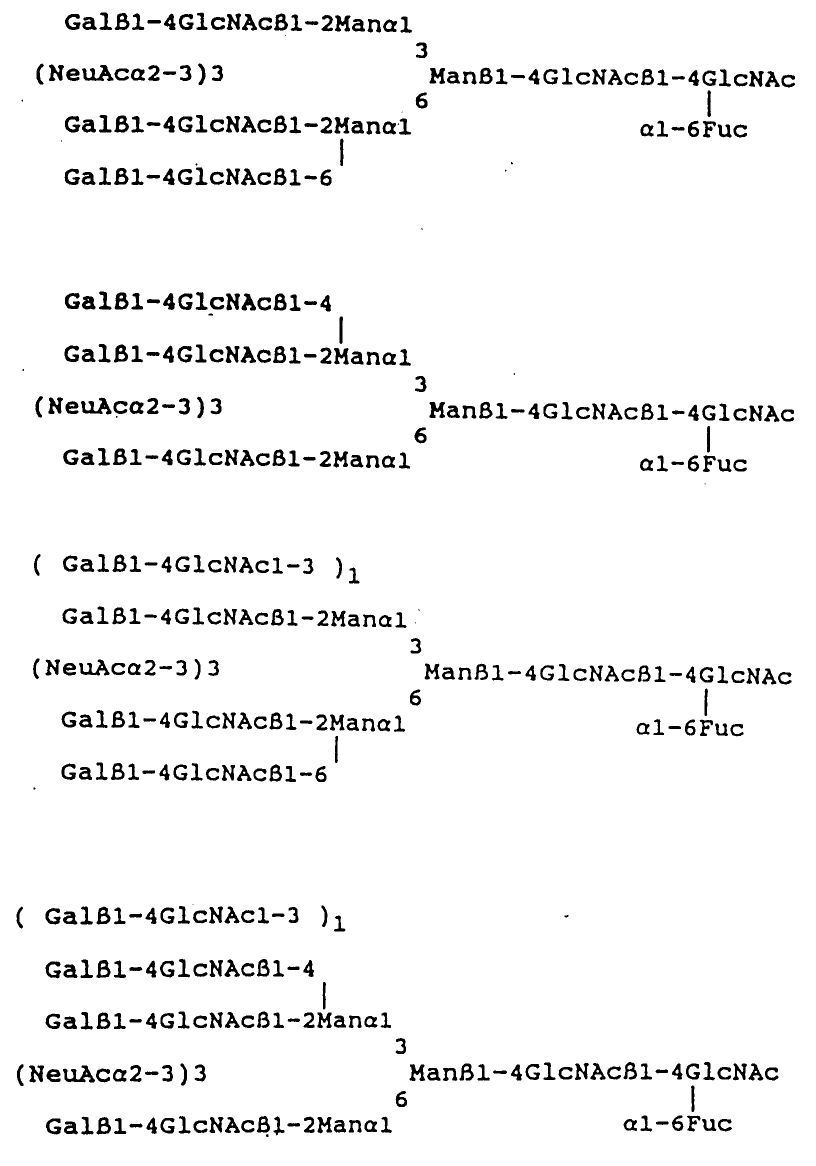 wobei NeuAc auch für N-Glykolylneuraminsäure steht.

20. Verfahren nach einem der Ansprüche 1 oder 11 - 19, dadurch gekennzeichnet, daß es eine spezifische Aktivität von mindestens 200 x 10⁶ IU/mg aufweist.

## Claims (Claims for the following Contracting State(s): BE, CH, LI, DE, DK, FR, GB, IT, LU, MC, NL, PT, SE)

1. Recombinant human-IFN-beta from CHO-cell cultures, characterised in that it contains a proportion of diantennal oligosaccharide structures amounting to at least 70%, triantennal oligosaccharide structures amounting to at least 20% and tetraantennal oligosaccharide structures amounting to between 0 and 5% and a sialic acid content of at least 90%.

2. Recombinant human IFN-beta according to Claim 1, characterised in that the proportion of diantennal oligosaccharide structures amounts to at least 75%.

3. Recombinant human IFN-beta according to Claim 1 or 2, characterised in that the proportion of triantennal oligosaccharide amounts to at least 25%.

4. Recombinant human-IFN-beta according to Claims 1 to 3, characterised in that the triantennal structures may comprise at least one N-acetyllactosamine repeat.

5. Recombinant human-IFN-beta according to Claims 1 to 4, characterised in that the triantennal structure is linked at positions 1->4 and/or 1->6.

6. Recombinant human-IFN-beta according to Claims 1 to 5, characterised in that the proportion of tetraantennal oligosaccharide structures amounts to 0.5 to 3%.

7. Recombinant human-IFN-beta according to one of Claims 1 to 6, characterised in that the sialic acid content is composed of N-acetylneuraminic acid and N-glycolylneuraminic acid.

8. Recombinant human-IFN-beta according to Claim 7, characterised in that the proportion of N-acetylneuraminic acid present is between 90 and 100% and the proportion of N-glycolylneuraminic acid amounts to 0 to 10%.

9. Recombinant human-IFN-beta according to Claims 1 to 8, characterised in that the recombinant IFN-beta also contains fucose at a rate of at least 85%, preferably 90% and most preferably > 95%.

10. Recombinant human-IFN-beta according to Claims 1 to 9, characterised in that at least one oligosaccharide structure corresponds to one of the following formulae: 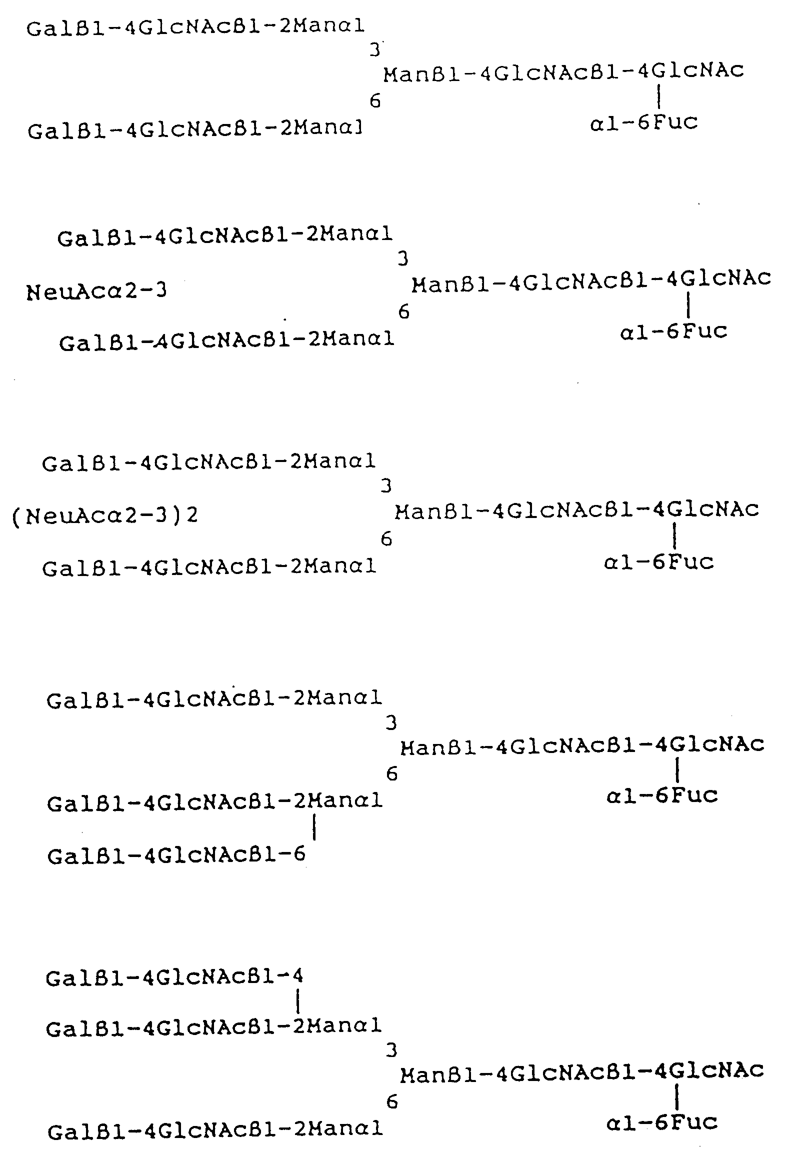 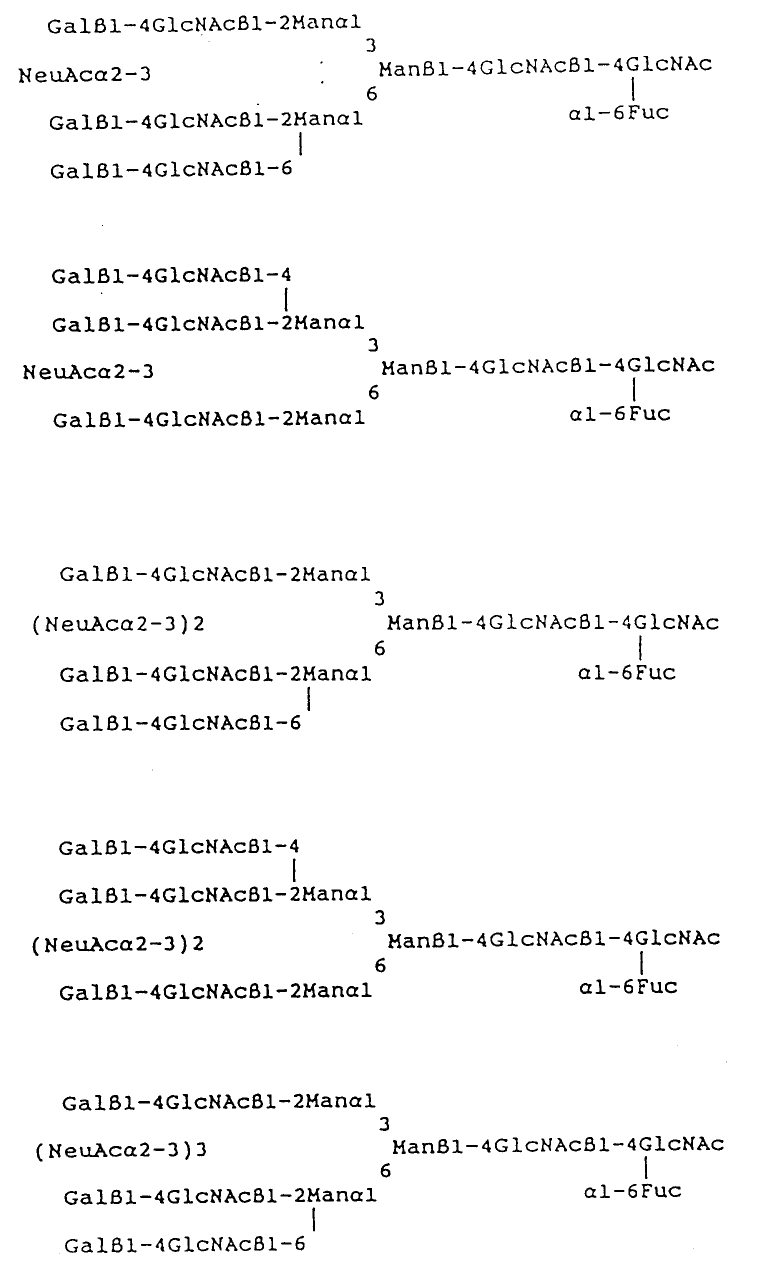 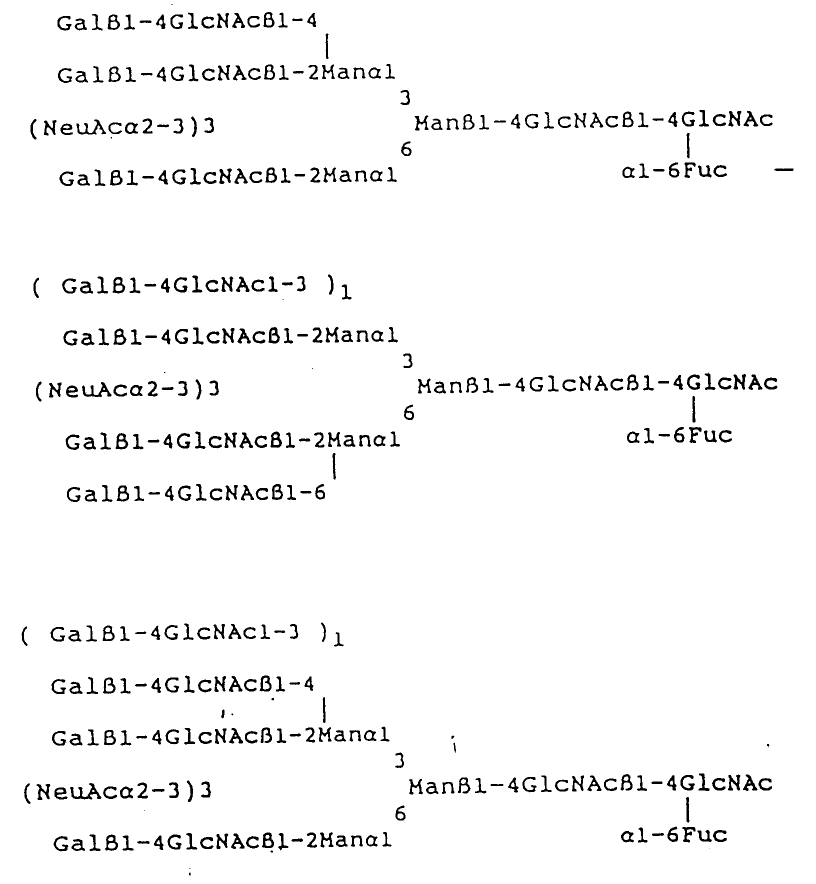 in which NeuAc represents N-glycolylneuraminic acid.

11. Recombinant human-IFN-beta according to Claims 1 to 10, characterised in that it has a specific activity of at least 200 x 10⁶ IU/mg.

12. Method for the production of recombinant human-IFN-beta according to Claims 1 to 11, characterised in that an aqueous solution of human-IFN-beta in an impure state is obtained by liquid/liquid phase extraction, then colour affinity chromatography performed, and finally metal chelation chromatography, followed by size-exclusion chromatography, in which the chromatographic stages are carried out in this order.

13. Method according to Claim 12, characterised in that the liquid/liquid phase extraction is carried out with an aqueous two-phase system based on polyalkyleneglycol/dextran or polyalkyleneglycol/salt.

14. Method according to Claim 13, characterised in that the polyalkyleneglycol used is polyethyleneglycol with a molecular weight of 1 000 to 6 000 or polypropyleneglycol with a molecular weight of 1 500 to 4 000.

15. Method according to Claim 13, characterised in that the salt used is NaCl, LiCl, NaI, KI, NA₂SO₄, NA₂HPO₄, K₂HPO₄,.K₂SO_{4,} NaH₂PO₄, KCl, NH₄Cl, (NH₄)₂SO₄, sodium citrate or sodium oxalate.

16. Method according to Claim 13, characterised in that the dyestuff used in affinity chromatography is Cibacron blue F 3 GA.

17. Method according to Claim 11, characterised in that the ions used in metal chelation chromatography are Cu²⁺, Zn²⁺, Co²⁺ or Ni²⁺.

18. Method according to Claim 12, characterised in that, for the size exclusion chromatography, separation media are used which have a separation range of 1 000 to 600 000 daltons, preferably Sephadex® G150, Sephadex® G150 superfine, Sephacryl® S-200 High Resolution, Superose 12 prep grade or TSK-SW 3000.

19. Method according to Claims 13 and 16, characterised in that the elution in affinity chromatography is carried out using PBS containing 10 to 70% by weight of ethylene glycol and/or 20 to 50% by weight of propylene glycol.

20. Method according to Claims 13 and 17, characterised in that the elution in metal chelation chromatography is carried out using a competitive substance such as imidazole, histidine, glycine or NH₄Cl or a pH gradient of around pH 7 to around pH2 or by means of isocratic elution with descending pH.

21. Method according to Claims 13 and 18, characterised in that the elution in size exclusion chromatography is carried out with PBS containing 0 to 0.5 mol/l NaCl.

22. Pharmaceutical preparation containing recombinant human-IFN-beta according to Claims 1 to 13, in a compound formulated with the usual vehicles and excipients.

23. Pharmaceutical preparation according to Claim 22, characterised in that it contains at least 25 x 10⁶ IU/ml IFN-beta.

24. Pharmaceutical formulation according to Claim 22 or 23, characterised in that it is available in forms appropriate for topical or parenteral administration.

25. Pharmaceutical formulation according to Claim 23 characterised in that it is available in the form of a gel, emulsion or ointment.

26. Pharmaceutical preparation according to Claim 23, characterised in that it is available in the form of a lyophilisate, solution or infusion.

## Claims (Claims for the following Contracting State(s): AT, ES, GR)

1. Method for the production of recombinant human-IFN-beta from CHO-cell cultures with a proportion of diantennal oligosaccharide structures amounting to at least 70%, triantennal oligosaccharide structures amounting to at least 20% and tetraantennal oligosaccharide structures amounting to between 0 and 5% and a sialic acid content of at least 90% characterised in that an aqueous solution of human-IFN-beta in an impure state is obtained by liquid/liquid phase extraction, then colour affinity chromatography performed, and finally metal chelation chromatography, followed by size-exclusion chromatography, in which the chromatographic stages are carried out in this order.

2. Method according to Claim 1, characterised in that the liquid/liquid phase extraction is carried out with a two-phase system on the basis of polyalkyleneglycol/dextran or polyalkyleneglyol/salt.

3. Method according to Claim 2, characterised in that the polyalkyleneglycol used is polyethyleneglycol with a molecular weight of 1 000 to 6000 or polypropylene glycol with a molecular weight of 1 500 to 4 000.

4. Method according to Claim 2, characterised in that the salt used is NaCl, LiCl, NaI, KI, NA₂SO₄, NA₂HPO₄, K₂HPO₄, K₂SO₄, NaH₂PO₄, KCl, NH₄Cl, (NH₄)₂SO₄, sodium citrate or sodium oxalate.

5. Method according to Claim 2, characterised in that the dyestuff used in affinity chromatography is Cibacron Blue F 3 GA.

6. Method according to Claim 1, characterised in that the ions used in metal chelation chromatography are Cu²⁺, Zn²⁺, Co²⁺ or Ni²⁺.

7. Method according to Claim 1 characterised in that, for the size exclusion chromatography, separation media are used which have a separation range of 1 000 to 600 000 daltons, preferably Sephadex® G150, Sephadex® G150 superfine, Sephacryl® S-200 High Resolution, Superose 12 prep grade or TSK-SW 3000.

8. Method according to one of the Claims 2 and 5, characterised in that the elution in affinity chromatography is carried out using PBS containing 10 to 70% by weight of ethylene glycol and/or 20 to 50% by weight of propylene glycol.

9. Method according to one of the Claims 2 and 6, characterised in that the elution in metal chelation chromatography is carried out using a competitive substance such as imidazole, histidine, glycine or NH₄Cl or a pH gradient of around pH 7 to around pH2 or by means of isocratic elution with descending pH.

10. Method according to one of the Claims 2 and 7, characterised in that the elution in size exclusion chromatography is carried out with PBS containing 0 to 0.5 mol/l NaCl.

11. Method according to Claim 1, characterised in that the proportion of diantennal oligosaccharide structures amounts to at least 75%.

12. Method according to Claim 1 or 11, characterised in that the proportion of triantennal oligosaccharide structures amounts to at least 25%.

13. Method according to one of the Claims 1 or 11 to 12, characterised in that the triantennal structures may contain at least one N-acetyllactosamine repeat.

14. Method according to Claim 1 or 11 to 13, characterised in that the triantennal structure is linked at positions 1->4 or 1->6.

15. Method according to one of the Claims 1 or 11 to 14, characterised in that the proportion of tetraantennal oligosaccharide structures is 0.5 to 3%.

16. Method according to one of the Claims 1 or 11 to 15, characterised in that the sialic acid is composed of N-acetylneuraminic acid and N-glycolylneuraminic acid.

17. Method according to Claim 16 characterised in that the proportion of n-acetylneuraminic acid is between 90 and 100% and the proportion of N-glycolylneuraminic acid is between 0 and 10%.

18. Method according to one of Claims 1 or 11 to 17, characterised in that the recombinant IFN-beta also has a fucose content of at least 85%, preferably 90% and most preferably > 95%.

19. Method according to one of Claims 1 or 11-18, characterised in that at least one oligosaccharide structure corresponds to the following formulae: 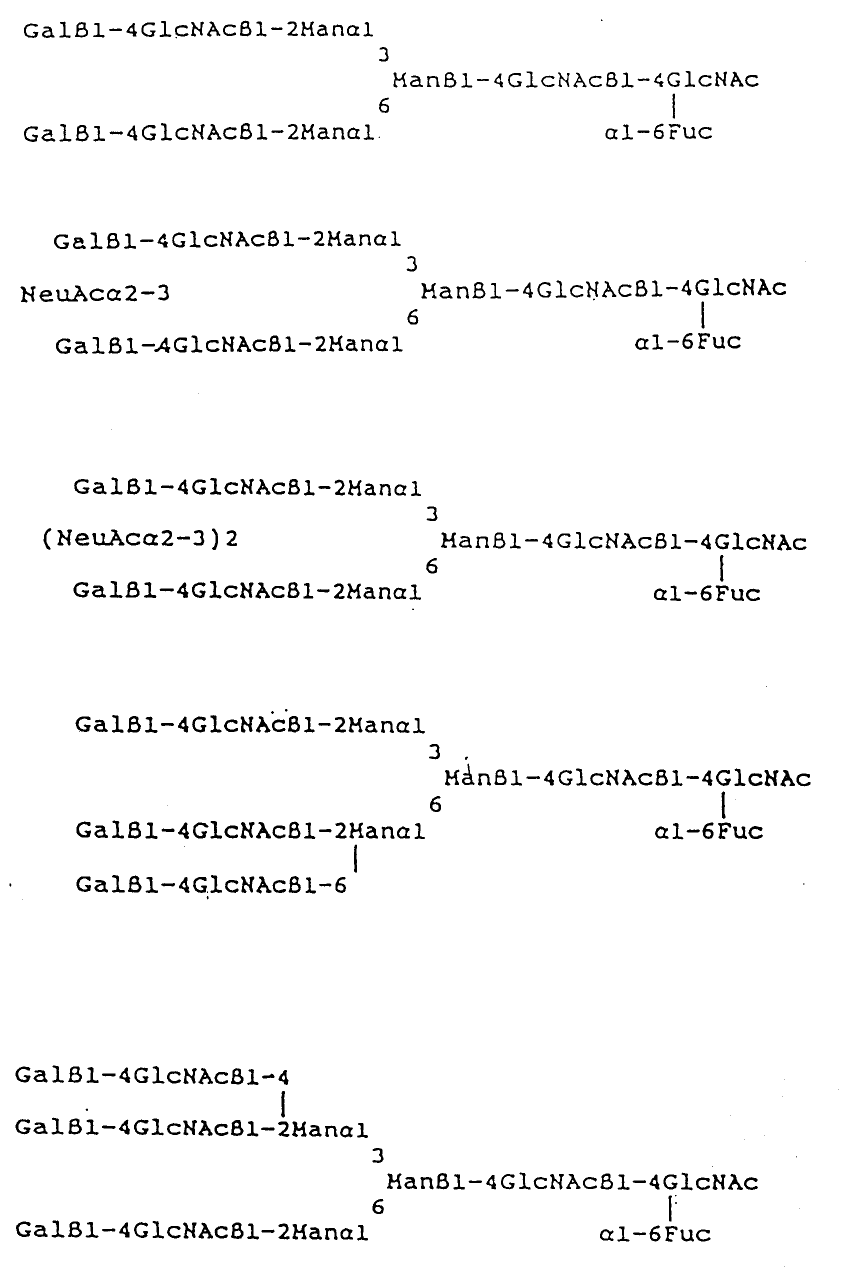 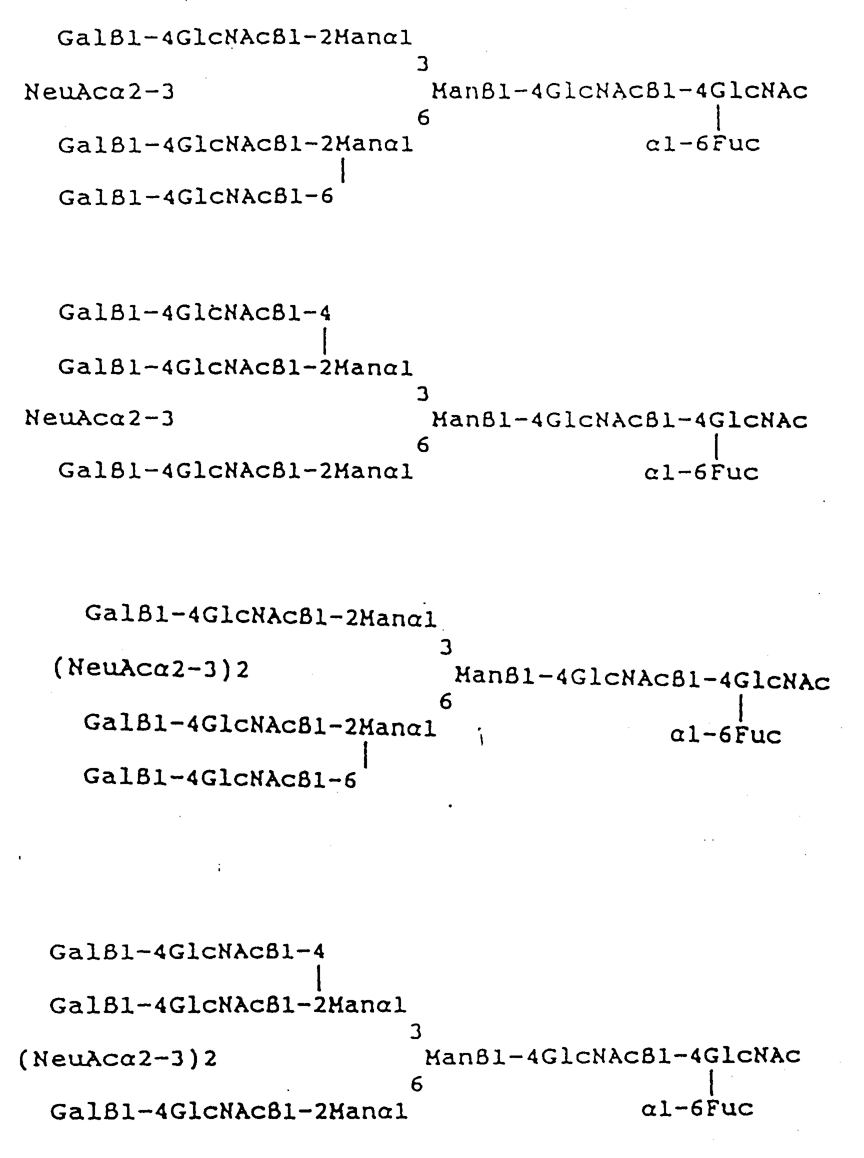 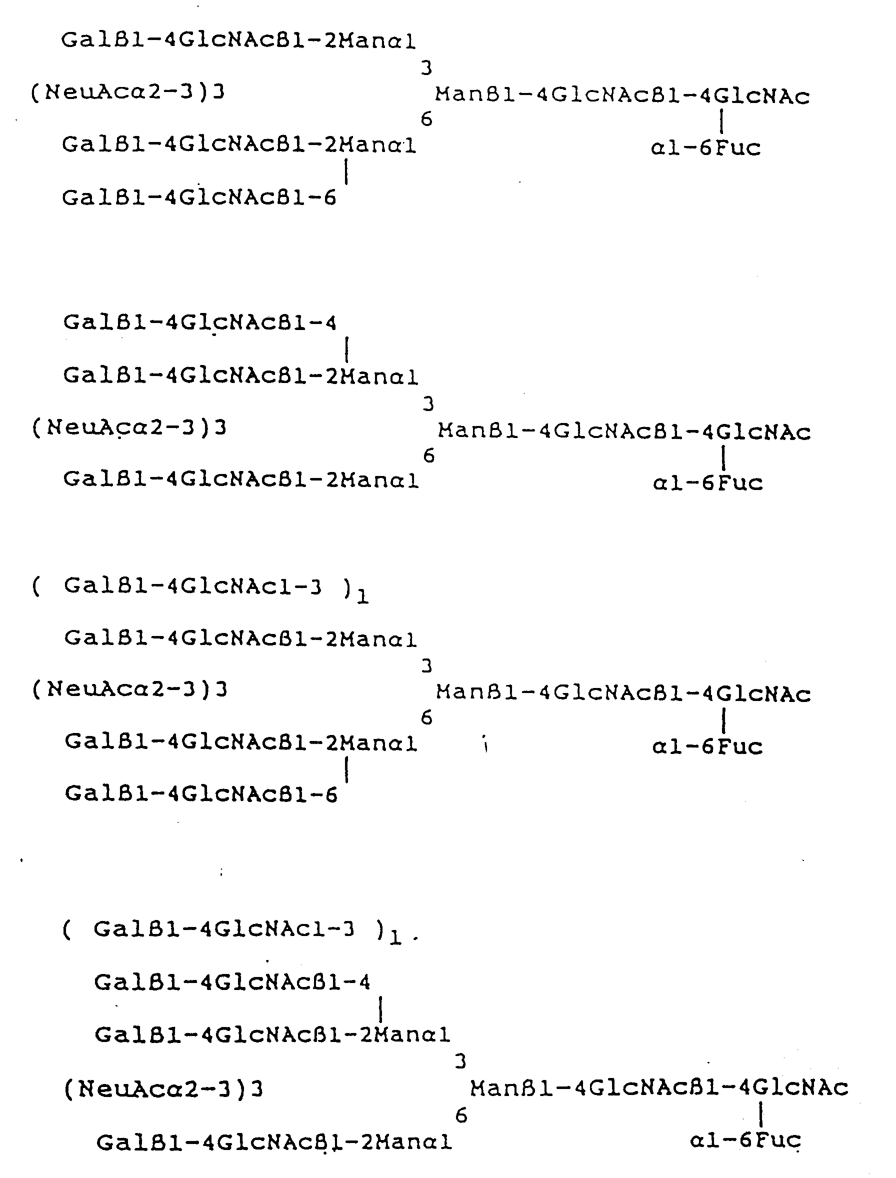 in which NeuAc also represents N-glycolylneuraminic acid.

20. Method according to Claims 1 or 11-19, characterised in that it displays a specific activity of at least 200 x 10⁶ IU/mg.

## Revendications (Revendications pour l'(les) Etat(s) contractant(s) suivant(s): BE, CH, LI, DE, DK, FR, GB, IT, LU, MC, NL, PT, SE)

1. IFN β humain recombinant de cultures de cellules CHO, caractérisé en ce que,
il possède une proportion de structures oligosaccharidiques biantennaires d'au moins 70 %, une proportion de structures oligosaccharidiques triantennaires d'au moins 20 % et une proportion de structures oligosaccharidiques tétraantennaires de 0 à 5 %, ainsi qu'une teneur en acide sialique d'au moins 90 %.

2. IFN β humain recombinant selon la revendication 1, caractérisé en ce que la proportion de structures oligosaccharidiques biantennaires est d'au moins 75 %.

3. IFN β humain recombinant selon la revendication 1 ou 2, caractérisé en ce que la proportion de structures oligosaccharidiques triantennaires est d'au moins 25 %.

4. IFN β humain recombinant selon l'une des revendications 1 à 3, caractérisé en ce que les structures triantennaires présentent, le cas échéant, au moins un motif répété N-acétyllactosamine.

5. IFN β humain recombinant selon les revendications 1 à 4, caractérisé en ce que la structure triantennaire est liée en 1→4 et/ou 1→6.

6. IFN β humain recombinant selon l'une des revendications 1 à 5, caractérisé en ce que la proportion de structures oligosaccharidiques tétraantennaires est de 0,5 à 3 %.

7. IFN β humain recombinant selon l'une des revendications 1 à 6, caractérisé en ce que la fraction acide sialique est constituée d'acide N-acétylneuraminique et d'acide N-glycolylneuraminique.

8. IFN β humain recombinant selon la revendication 7, caractérisé en ce que la proportion d'acide N-acétylneuraminique est de 90 à 100 % et la proportion d'acide N-glycolylneuraminique est de 0 à 10 %.

9. IFN β humain recombinant selon l'une des revendications 1 à 8, caractérisé en ce que l'IFN β recombinant a en outre une teneur en fucose d'au moins 85 %, de préférence 90 %, et de façon particulièrement préférée >95 %.

10. IFN β humain recombinant selon l'une des revendications 1 à 9, caractérisé en ce que au moins une structure oligosaccharidique correspond à l'une des formules suivantes : 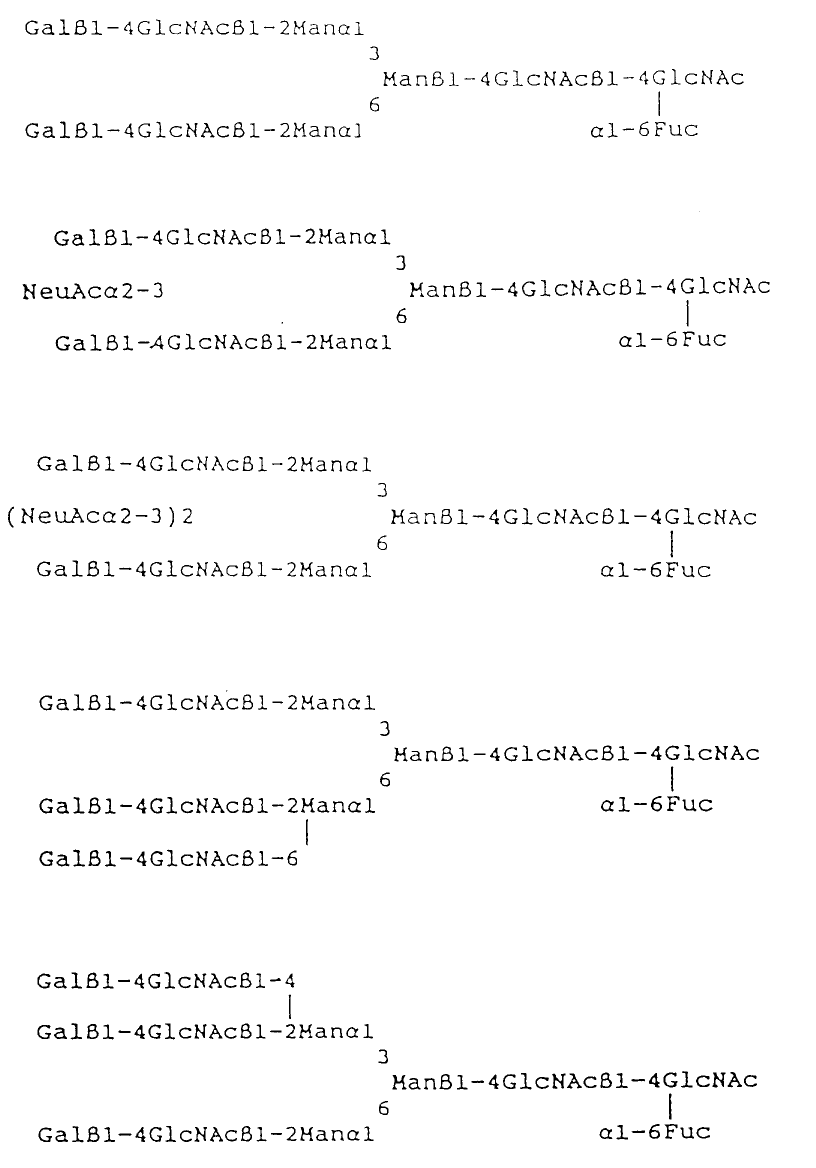 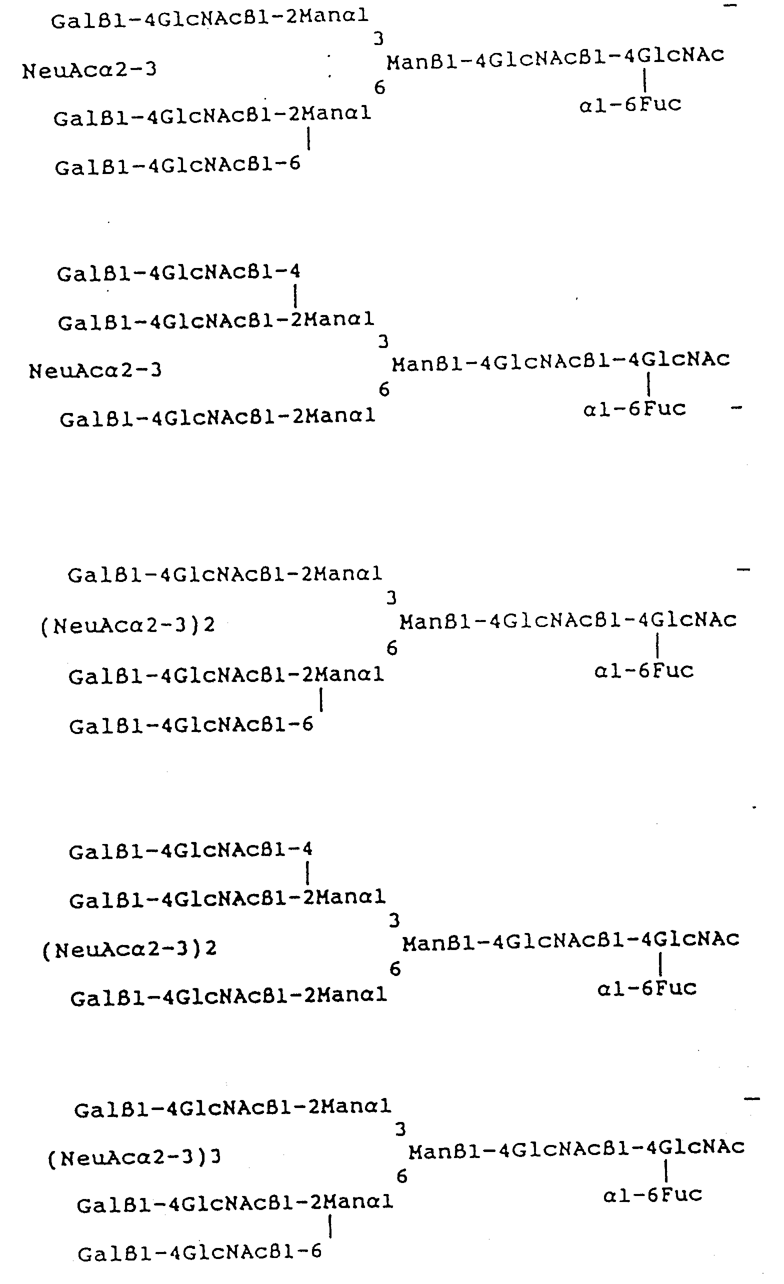 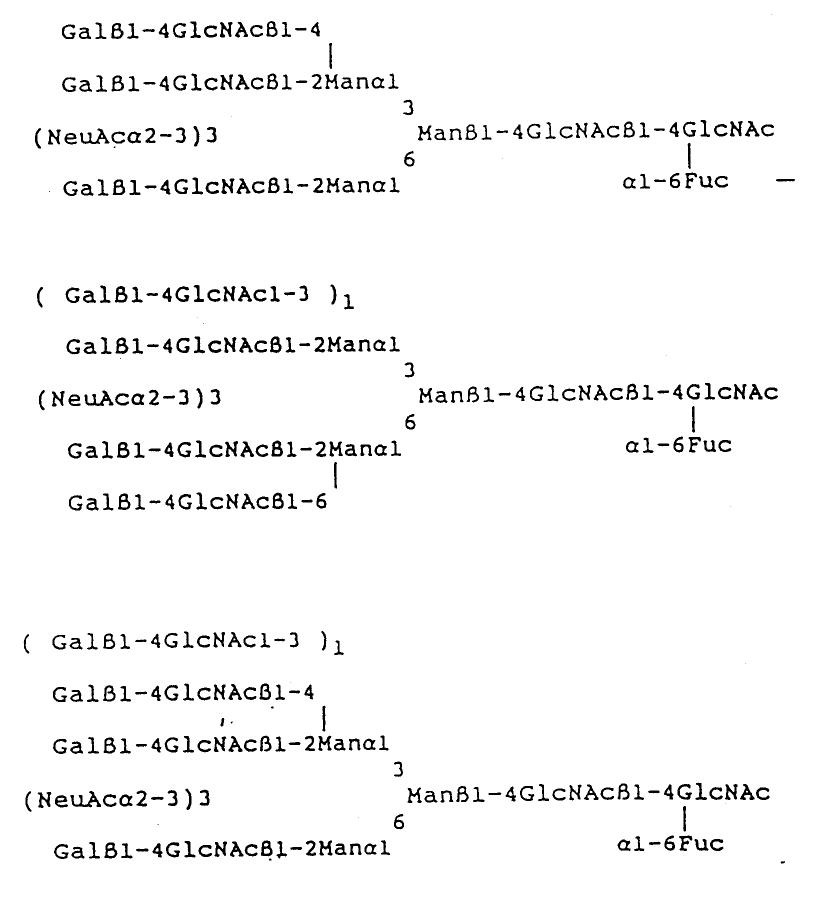 dans laquelle NeuAc signifie acide N-glycolylneuraminique.

11. IFN β humain recombinant selon l'une des revendications 1 à 10, caractérisé en ce qu'il possède une activité spécifique d'au moins 200 x 10⁶ UI/mg.

12. Procédé de fabrication d'IFN β humain recombinant selon l'une des revendications 1 à 11, caractérisé en ce qu'une solution aqueuse d'IFN β humain sous forme non purifiée est enrichie par une extraction de phase liquide/liquide, puis est soumise à une chromatographie d'affinité par couplage de colorants, qu'ensuite on effectue une chromatographie d'affinité par chélation de métaux, laquelle est suivie d'une chromatographie par perméation de gel, les différentes étapes chromatographiques étant réalisées dans cet ordre.

13. Procédé selon la revendication 12, caractérisé en ce que l'extraction de phase liquide/liquide est effectuée avec un système aqueux à deux phases à base de polyalkylèneglycol/dextran ou polyalkylèneglycol/sel.

14. Procédé selon la revendication 13, caractérisé en ce que le polyalkylèneglycol utilisé est le polyéthylèneglycol avec une masse moléculaire de 1 000 à 6 000 ou le polypropylèneglycol avec une masse moléculaire de 1 500 à 4 000.

15. Procédé selon la revendication 13, caractérisé en ce que le sel utilisé est NaCl, LiCl, NaI, KI, Na₂SO₄, Na₂HPO₄, K₂HPO₄, K₂SO₄, NaH₂PO₄, KCl, NH₄Cl, (NH₄)₂SO₄, citrate de sodium ou oxalate de sodium.

16. Procédé selon la revendication 13, caractérisé en ce que le colorant dans la chromatographie d'affinité est le bleu Cibacron F3 GA.

17. Procédé selon la revendication 12, caractérisé en ce que dans la chromatographie par chélation de métaux, on utilise des ions Cu²⁺, Zn²⁺, Co²⁺ ou Ni²⁺.

18. Procédé selon la revendication 12, caractérisé en ce que, pour la chromatographie par perméation de gel, on utilise des milieux de séparation, qui ont un domaine de séparation de 1 000 daltons à 600 000 daltons, de préférence, Sephadex® G150, Sephadex® G150 superfine Sephacryl® S-200 à haute résolution, Superose 12 qualité préparative ou TSK-SW 3000.

19. Procédé selon l'une des revendications 13 et 16, caractérisé en ce que l'on effectue l'élution en chromatographie d'affinité avec PBS contenant 10 à 70 % en poids d'éthylèneglycol et/ou 20 à 50 % en poids de propylèneglycol.

20. Procédé selon l'une des revendications 13 et 17, caractérisé en ce que l'élution en chromatographie par chélation de métaux est réalisée avec des substances compétitives telles que l'imidazole, l'histidine, la glycine ou NH₄Cl ou un gradient de pH d'environ pH 7 à environ pH 2 ou par une élution isocratique avec des valeurs de pH décroissantes.

21. Procédé selon l'une des revendications 13 et 18, caractérisé en ce que l'on effectue l'élution en chromatographie par perméation de gel avec PBS contenant 0 à 0,5 mole/l de NaCl.

22. Préparation pharmaceutique contenant un IFN β humain recombinant selon l'une des revendications 1 à 13 en association avec les véhicules et substances auxiliaires habituels.

23. Préparation pharmaceutique selon la revendication 22, caractérisée en ce qu'elle contient au moins 25 x 10⁶ UI/ml d'IFN β.

24. Formulation pharmaceutique selon la revendication 22 ou 23, caractérisée en ce que elle est sous une forme adaptée à une administration topique ou parentérale.

25. Formulation pharmaceutique selon la revendication 23, caractérisée en ce qu'elle est sous la forme d'un gel, d'une émulsion ou d'un sel.

26. Préparation pharmaceutique selon la revendication 23, caractérisée en ce qu'elle est sous la forme d'un lyophilisat, d'une solution ou d'une perfusion.

## Revendications (Revendications pour l'(les) Etat(s) contractant(s) suivant(s): AT, ES, GR)

1. Procédé de fabrication d'IFN β humain recombinant de cultures de cellules CHO avec une proportion de structures oligosaccharidiques biantennaires d'au moins 70 %, une proportion de structures oligosaccharidiques triantennaires d'au moins 20 % et une proportion de structures oligosaccharidiques tétraantennaires de 0 à 5 %, ainsi qu'une teneur en acide sialique d'au moins 90 %, caractérisé en ce qu'une solution aqueuse d'IFN β humain sous forme non purifiée est enrichie par une extraction de phase liquide/liquide, puis est soumise à une chromatographie d'affinité par couplage de colorants, qu'ensuite on effectue une chromatographie d'affinité par chélation de métaux, laquelle est suivie d'une chromatographie par perméation de gel, les différentes étapes chromatographiques étant réalisées dans cet ordre.

2. Procédé selon la revendication 1, caractérisé en ce que l'extraction de phase liquide/liquide est effectuée avec un système aqueux à deux phases à base de polyalkylèneglycol/dextran ou polyalkylèneglycol/sel.

3. Procédé selon la revendication 2, caractérisé en ce que le polyalkylèneglycol utilisé est le polyéthylènegycol avec une masse moléculaire de 1 000 à 6 000 ou le polypropylèneglycol avec une masse moléculaire de 1 500 à 4 000.

4. Procédé selon la revendication 2, caractérisé en ce que le sel utilisé est NaCl, LiCl, NaI, KI, Na₂SO₄, Na₂HPO₄, K₂HPO₄, K₂SO₄, NaH₂PO₄, KCl, NH₄Cl, (NH₄)₂SO₄, citrate de sodium ou oxalate de sodium.

5. Procédé selon la revendication 2, caractérisé en ce que le colorant dans la chromatographie d'affinité est le bleu Cibacron F3 GA.

6. Procédé selon la revendication 1, caractérisé en ce que dans la chromatographie par chélation de métaux, on utilise des ions Cu²⁺, Zn²⁺, Co²⁺ ou Ni²⁺.

7. Procédé selon la revendication 1, caractérisé en ce que, pour la chromatographie par perméation de gel, on utilise des milieux de séparation qui ont un domaine de séparation de 1 000 daltons à 600 000 daltons, de préférence, Sephadex® G150, Sephadex® G150 superfine, Sephacryl® S-200 à haute résolution, Superose 12 prepgrade ou TSK-SW 3000.

8. Procédé selon l'une des revendications 2 et 5, caractérisé en ce que l'on effectue l'élution en chromatographie d'affinité avec PBS contenant 10 à 70 % en poids d'éthylèneglycol et/ou 20 à 50 % en poids de propylèneglycol.

9. Procédé selon l'une des revendications 2 et 6, caractérisé en ce que l'élution en chromatographie par chélation de métaux est réalisée avec des substances compétitives telles que l'imidazole, l'histidine, la glycine ou NH₄Cl ou un gradient de pH d'environ pH 7 à environ pH 2 ou par une élution isocratique avec des valeurs de pH décroissantes.

10. Procédé selon l'une des revendication 2 et 7, caractérisé en ce que l'élution en chromatographie par perméation de gel est effectuée avec PBS contenant 0 à 0,5 mole/l de NaCl.

11. Procédé selon la revendication 1, caractérisé en ce que la proportion de structures oligosaccharidiques biantennaires est d'au moins 75 %.

12. Procédé selon la revendication 1 ou 11, caractérisé en ce que la proportion de structures oligosaccharidiques triantennaires est d'au moins 25 %.

13. Procédé selon l'une des revendications 1 ou 11-12, caractérisé en ce que les structures triantennaires présentent, le cas échéant, au moins un motif répété N-acétyllactosamine.

14. Procédé selon les revendications 1 ou 11-13, caractérisé en ce que la structure triantennaire est liée en 1→4 et/ou 1→6.

15. Procédé selon l'une des revendications 1 ou 11-14, caractérisé en ce que la proportion de structures oligosaccharidiques tétraantennaires est de 0,5 à 3 %.

16. Procédé selon l'une des revendications 1 ou 11-15, caractérisé en ce que la fraction acide sialique est constituée d'acide N-acétylneuraminique et d'acide N-glycolylneuraminique.

17. Procédé selon la revendication 16, caractérisé en ce que la proportion d'acide N-acétylneuraminique est de 90 à 100 % et la proportion d'acide N-glycolylneuraminique est de 0 à 10 %.

18. Procédé selon l'une des revendications 1 ou 11-17, caractérisé en ce que l'IFN β recombinant a en outre une teneur en fucose d'au moins 85 %, de préférence 90 %, et de façon particulièrement préférée >95 %.

19. Procédé selon l'une des revendications 1 ou 11-18, caractérisé en ce que au moins une structure oligosaccharidique correspond à l'une des formules suivantes : 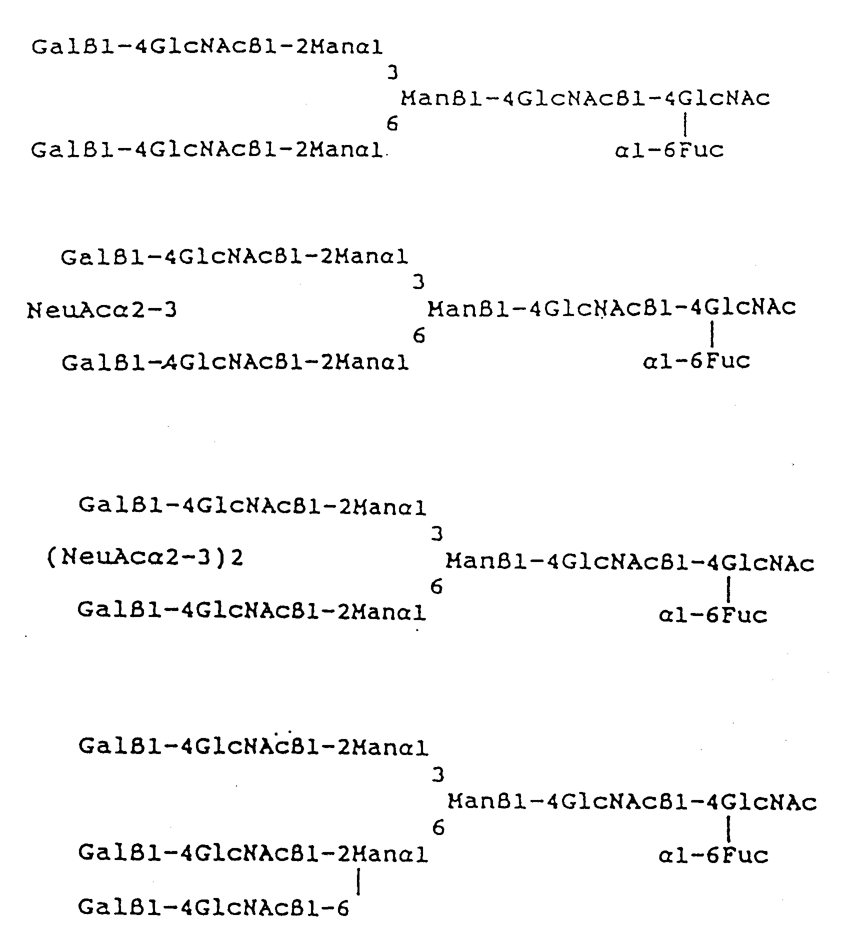 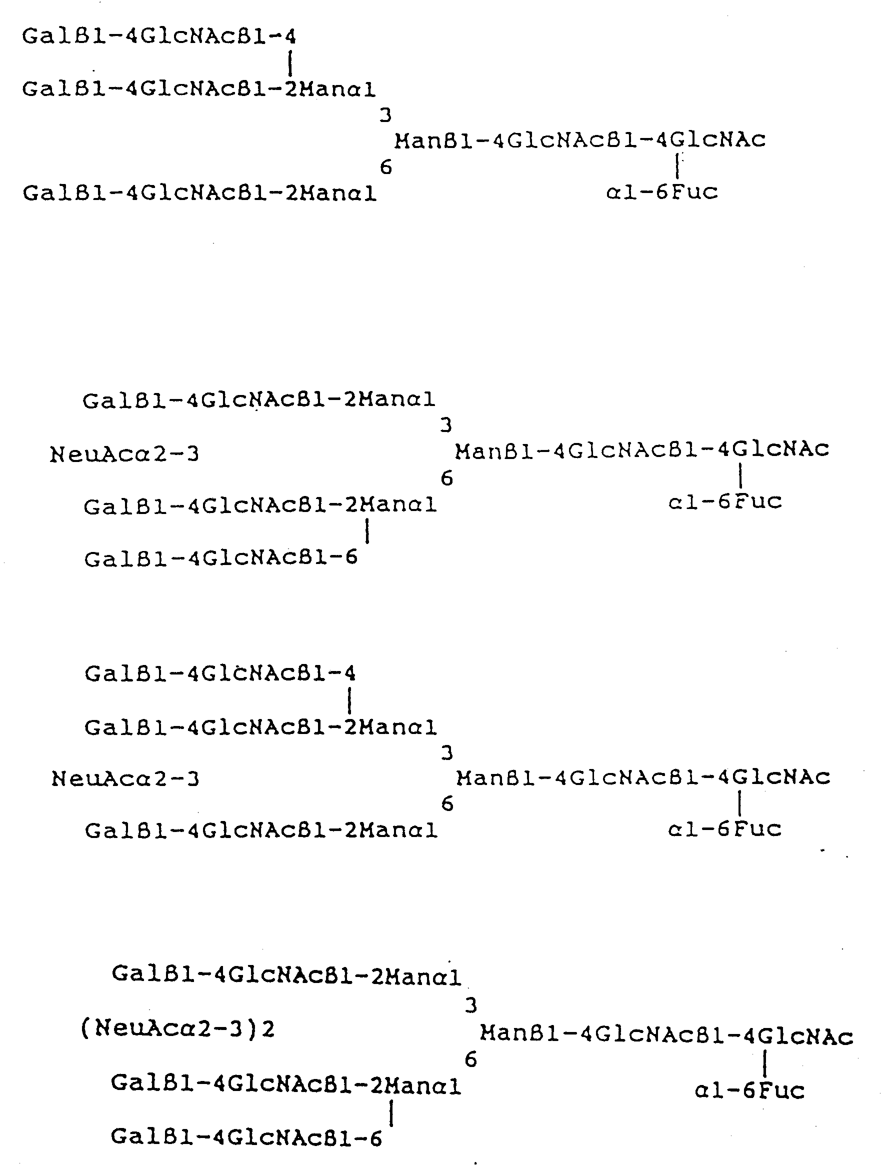 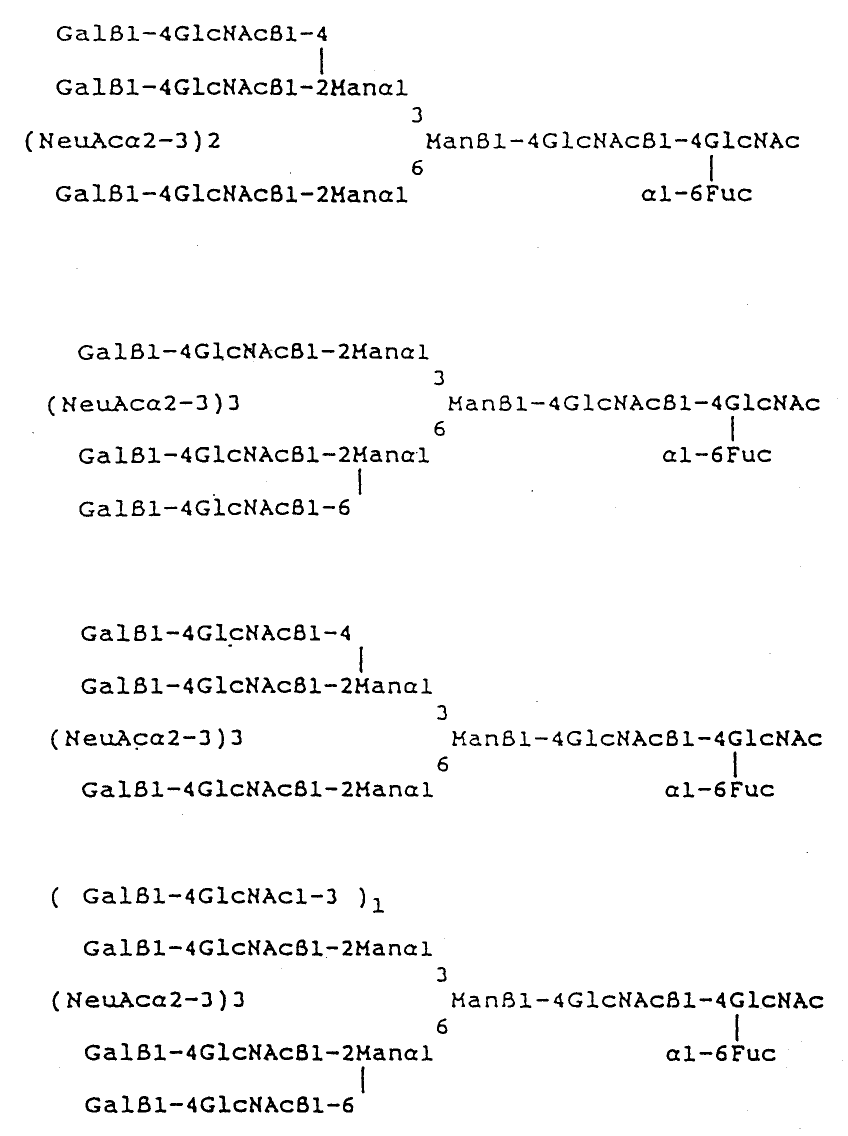 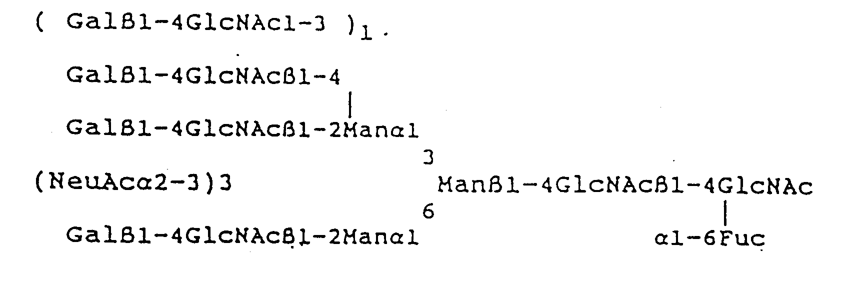 dans laquelle NeuAc signifie acide N-glycolylneuraminique.

20. Procédé selon l'une des revendications 1 ou 11-19, caractérisé en ce que l'activité spécifique est d'au moins 200 x 10⁶ UI/mg.
